(19) 

(11) **EP 3 738 577 B1**

(12) **EUROPÄISCHE PATENTSCHRIFT**

(45) Veröffentlichungstag und Bekanntmachung des Hinweises auf die Patenterteilung:
**23.09.2026 Patentblatt 2026/39**

(21) Anmeldenummer: **20176997.3**

(22) Anmeldetag: **13.04.2017**

(51) Internationale Patentklassifikation (IPC):
***A61K 8/26*** *(2006.01)* ***C09C 1/64*** *(2006.01)*
***C09C 1/00*** *(2006.01)* ***C09C 1/62*** *(2006.01)*
***C09C 1/66*** *(2006.01)*

(52) Gemeinsame Patentklassifikation (CPC):
**C09C 1/644; A61K 8/0254; A61K 8/26; A61K 8/55; A61K 8/72; A61K 8/8117; A61Q 3/02; C09C 1/0015; C09C 1/627; C09C 1/642; C09C 1/648; C09C 1/66;** A61K 2800/412; A61K 2800/612; C01P 2004/61; (Forts.)

(54) **OBERFLÄCHENMODIFIZIERTE EFFEKTPIGMENTE**
SURFACE-MODIFIED EFFECT PIGMENTS
PIGMENTS À EFFET MODIFIÉ EN SURFACE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2016 EP 16000855**

(43) Veröffentlichungstag der Anmeldung:
**18.11.2020 Patentblatt 2020/47**

(62) Dokumentnummer(n) der früheren Anmeldung(en) nach Art. 76 EPÜ:
**17716285.6 / 3 442 491**

(73) Patentinhaber: **ECKART GmbH**
**91235 Hartenstein (DE)**

(72) Erfinder:
- **PIPPINGER, Christina**
  **91275 Auerbach (DE)**
- **GEBHARD, Ann-Katrin**
  **91235 Hartenstein (DE)**
- **SCHILLING, Christine**
  **91235 Hartenstein (DE)**
- **SCHMIDT, Ulrich**
  **91235 Hartenstein (DE)**

(74) Vertreter: **Altana IP Department**
**Altana Management Services GmbH**
**Abelstraße 45**
**46483 Wesel (DE)**

(56) Entgegenhaltungen:
**DE-A1- 102005 036 333**
**DE-A1- 102007 034 928**
**DE-A1- 19 820 112**
**US-A1- 2010 047 199**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



**(Forts. nächste Seite)**

(52) Gemeinsame Patentklassifikation (CPC): (Forts.)
C01P 2006/60; C09C 2200/102; C09C 2200/301;
C09C 2220/10

## Beschreibung

Die vorliegende Erfindung betrifft ein oberflächenmodifiziertes Effektpigment sowie ein Verfahren zur Herstellung desselben sowie Nagellackkompositionen enthaltend dieses oberflächenmodifizierte Effektpigment.

EP 1 812 518 A2 offenbart Perlglanzpigmente, welche an der Oberfläche mit wenigstens einer phosphorhaltigen Verbindung versehen sind. Die Perlglanzpigmente eignen sich insbesondere für die Verwendung in Pulverlacken.

EP 2 227 508 A1 beschreibt mit mindestens einer Metalloxidschicht beschichtete Metalleffektpigmente, wobei die Oberfläche der Metalloxidschicht mindestens ein fluoralkyl- und/oder fluorarylhaltiges Oberflächenmodifizierungsmittel bzw. kovalent gebundenes Polysiloxan aufweist. Die Metalleffektpigmente kommen insbesondere in Pulverlacken zu Anwendung.

EP 2 318 463 A1 offenbart mit mindestens einer Metalloxidschicht beschichtete Metalleffektpigmente, wobei die Oberfläche der Metalloxidschicht kovalent gebundenes Polysiloxan aufweist. Die Metalleffektpigmente eignen sich insbesondere zur Verwendung in Pulverlacken.

EP 2 576 702 A1 betrifft die Verwendung oberflächenmodifizierter Effektpigmente insbesondere in Pulverlacken. Die Effektpigmentoberfläche ist hier mit wenigstens einer epoxidgruppenhaltigen Verbindung oberflächenmodifiziert.

EP 1 462 085 A1 offenbart eine Nagellackkomposition mit Spiegeleffekt, welche Partikel mit metallischem Glanz in einem Anteil von $\geq$ 2 Gew.-%, bezogen auf das Gesamtgewicht der Nagellackkomposition, umfasst. EP 1 462 085 A1 offenbart keine oberflächenmodifizierten Effektpigmente.

EP 1 299 066 A2 beschreibt einen Aluminiumplättchen enthaltenden Nagellack mit spiegelartigem Aussehen. Gemäß EP 1 299 066 A2 muss der Nagellack als Filmbildner Nitrocellulose mit einem Molekulargewicht von > 56000 umfassen, damit ein spiegelartiger Effekt auf einem Fingernagel erzielt werden kann. Dennoch sind die hiermit erreichbaren Spiegeleffekte limitiert und verbesserungswürdig.

EP 1 746 913 A2 offenbart einen flexiblen Artikel umfassend wenigstens eine Klebeschicht zur Fixierung des Artikels auf einem Fingernagel, wenigstens einen organischen Film sowie wenigstens eine, für z.B. einen optischen Effekt verantwortliche Komponente. Letztere kann beispielsweise dazu dienen, dem flexiblen Artikel einen Spiegeleffekt zu verleihen.

EP 1 792 598 A1 beschreibt einen Nagellack mit Spiegelglanz, welcher kolloidale Edelmetallpartikel mit einer mittleren Partikelgröße von 10 bis 100 nm in einem Anteil von 5 bis 50 Gew.-%, bezogen auf das Gesamtgewicht des Nagellacks, enthält.

EP 1 796 794 A1 offenbart eine kosmetische Zusammensetzung, welche ein PVD-Aluminiumpigment in einer Pigmentierungshöhe von 0,05 bis 5,0 Gew.-%, bezogen auf das Gesamtgewicht der kosmetischen Zusammensetzung, und wenigstens ein leafing-Additiv umfasst. Als leafing-Additiv dienen langkettige Phosphorsäureester oder ein Gemisch aus mehreren langkettigen Phosphorsäureestern. In den meisten Nagellacken jedoch kommt der leafing-Effekt nicht voll zur Geltung.

EP 1 082 952 A1 offenbart eine kosmetische Zusammensetzung, beispielsweise einen Nagellack, welche metallbeschichtete Glaspartikel umfasst.

JP 2012081236 A offenbart die Befestigung eines Spiegels an einem Fingernagel.

EP 2 248 514 A2 beschreibt nitrocellulosefreie Nagellackkompositionen, welche wenigstens ein Styrol/Maleinsäureanhydrid-Copolymer als hochglänzenden Filmbildner, wenigstens ein Epoxyharz als Co-Filmbildner, wenigstens eine reaktive Komponente sowie wenigstens ein Lösungsmittel umfasst. Die nitrocellulosefreie Nagellackkomposition soll vergleichbare oder bessere Haftungseigenschaften als nitrocellulosehaltige Nagellackkompositionen aufweisen. In EP 2 248 514 A2 kommen keine oberflächenmodifizierten Effektpigmente als Farbmittel zum Einsatz.

Aufgabe der vorliegenden Erfindung ist es, ein Effektpigment für die Verwendung in einer Nagellackkomposition bereitzustellen, welches das optische Erscheinungsbild der Nagellackkomposition nach Applikation und Trocknung maßgeblich bestimmt.

Im Fall von hochwertigen Metalleffektpigmenten soll ein Spiegelglanz und bevorzugt ein besonderer Spiegelglanz ermöglicht werden.

Insbesondere ist es eine weitere Aufgabe der vorliegenden Erfindung, eine Nagellackkomposition bereitzustellen, die einen guten leafing Effekt oberflächenmodifizierter Effektpigmente ermöglicht.

Die der Erfindung zugrunde liegende Aufgabe wird durch die Bereitstellung eines oberflächenmodifizierten Effektpigments umfassend ein optional mit wenigstens einem Metalloxid, Metallhydroxid und/oder Metalloxidhydrat beschichtetes, metallisches plättchenförmiges Substrat, wobei

das metallische plättchenförmige Substrat durch PVD-Verfahren hergestellt wird und eine mittlere Dicke $h_{50}$ aus einem Bereich von 13 nm bis 60 nm aufweist und als Ausgangsmaterial (Additiv) zur Oberflächenmodifizierung

i) Phosphorsäurecetylester oder Cetylphosphorsäure aus einem Bereich von 10 Gew.-% bis 50 Gew.-% verwendet wird und das Aluminiumeffektpigment einen $D_{50}$-Wert aus einem Bereich von 2,5 $\mu$m bis 90 $\mu$m aufweist.

Die Aufgabe der Erfindung wird des Weiteren durch ein Verfahren zur Herstellung des oberflächenmodifizierten Effektpigments gemäß Anspruch 5 gelöst.

Weiterhin wird die Aufgabe der zugrundeliegenden Erfindung gelöst durch die Verwendung eines oberflächenmodifizierten Effektpigments nach Anspruch 1 oder 2 in Nagellacken.

Die erfindungsgemäß oberflächenmodifizierten Effektpigmente gemäß Anspruch 1 können ein optional mit wenigstens einem Metalloxid, Metallhydroxid und/oder Metalloxidhydrat beschichtetes, metallisches plättchenförmiges Substrat oder ein optional mit wenigstens einem Metalloxid, Metallhydroxid und/oder Metalloxidhydrat beschichtetes nichtmetallisches plättchenförmiges Substrat umfassen.

Die metallischen plättchenförmigen Substrate werden über PVD-Verfahren hergestellt.

Die vorgenannten metallischen plättchenförmigen Substrate können auch eine oder mehrere Schichten aus oder mit wenigstens einem hoch- und/oder niedrigbrechendem Metalloxid, Metallhydroxid und/oder Metalloxidhydrat aufweisen und optional getrocknet und/oder optional kalziniert sein. So können als metallische plättchenförmige Substrate also auch kommerziell erhältliche beschichtete Metalleffektpigmente verwendet werden. Gemäß einer bevorzugten Ausführungsform sind die erfindungsgemäß zu verwendenden metallischen, plättchenförmigen Substrate nicht beschichtet.

Erfindungsgemäß sind die metallischen plättchenförmigen Substrate Aluminium-PVD-Pigmente.

Diese weisen bevorzugt eine mittlere Dicke $h_{50}$ aus einem Bereich von 13 nm bis 60 nm und besonders bevorzugt aus einem Bereich von 20 nm bis 40 nm auf.

Unterhalb von 13 nm werden die metallischen PVD-Pigmente, insbesondere Aluminiumpigmente zu dunkel und zu transparent.

Oberhalb von 80 nm lassen die optischen Eigenschaften sowie die Deckkraft erheblich nach und ein Spiegelglanz ist nur schwer erreichbar.

Werden Metalleffektpigmente in kosmetischen Formulierungen eingesetzt, so müssen sie gewissen Reinheitsanforderungen genügen wie beispielsweise die EU Cosmetic Regulation 1223/2009 oder FDA 21CFR part 73.

Werden beispielsweise Aluminiumplättchen als metallisches plättchenförmiges Substrat eingesetzt, weisen diese bevorzugt einen Aluminiumgehalt von ≥ 97 Gew.-%, weiter bevorzugt von ≥ 98 Gew.-%, besonders bevorzugt von ≥ 99 Gew.-% und ganz besonders bevorzugt von ≥ 99,7 Gew.-%, jeweils bezogen auf das Gesamtgewicht des Aluminiumplättchens, auf. Bei einer bevorzugten Ausführungsform weisen die Aluminiumplättchen weiterhin einen Quecksilbergehalt von bevorzugt ≤ 1 ppm, einen Arsengehalt von bevorzugt ≤ 2 ppm, einen Bleigehalt von bevorzugt ≤ 10 ppm, einen Cadmiumgehalt von bevorzugt ≤ 1 ppm, einen Bariumgehalt von bevorzugt ≤ 10 ppm, einen Chromgehalt von bevorzugt ≤ 20 ppm, einen Nickelgehalt von bevorzugt ≤ 20 ppm, einen Kupfergehalt von bevorzugt ≤ 20 ppm, einen Cobaltgehalt von bevorzugt ≤ 20 ppm, einen Antimongehalt von bevorzugt ≤ 2 ppm, einen Selengehalt von bevorzugt ≤ 10 ppm und einen Zinkgehalt von bevorzugt ≤ 20 ppm auf.

Bei einer bevorzugten Ausführungsform umfassen die in der erfindungsgemäßen Nagellackkomposition zu verwendenden oberflächenmodifizierten Effektpigmente als metallisches plättchenförmiges Substrat durch PVD-Verfahren hergestellte Aluminiumplättchen oder Metalleffektpigmente gemäß dem Hauptanspruch der WO 2010/086165 A1, wie beispielsweise METALURE A41010 AE, METALURE A41506 EN, METALURE A 41510 EN, METALURE A31017 AE, METALURE A31010 BG, METALURE A31010 AE, METALURE A21010 AE, METALURE L55350AE, METALURE L63418, METALURE L 55700, METALURE A 61010 BG, METALURE A61010 AE, METALURE A61006 AE, METALURE A61006 BG, METALURE A41010 BG oder METALURE L71011AE, alle Fa. ECKART GmbH.

Unter der mittleren Dicke $h_{50}$ wird erfindungsgemäß das der Medianwert der Summenverteilungskurve der Dickenverteilung verstanden, sofern nicht anders angegeben.

Die Bestimmung von $h_{50}$ erfolgte bevorzugt gemäß der in der WO 2004/087816 A2 (Seiten 24 und 25) beschriebenen Methode mittels REM.

Der $h_{50}$-Wert der Summenhäufigkeitsverteilung der Dickenverteilungsfunktion, wie sie bevorzugt durch Auszählung im REM erhalten wird, gibt an, dass 50% der vermessenen oberflächenmodifizierten Effektpigmente eine Dicke aufweisen, der gleich oder kleiner als der jeweils angegebene Wert ist.

Bei weiter bevorzugten Ausführungsformen werden als metallisches plättchenförmiges Substrat nassvermahlene Aluminiumplättchen mit einem Span der Dickenverteilung $\Delta h = (h_{90}-h_{10})/h_{50}$ aus einem Bereich von 30 bis 140 % und bevorzugt aus einem Bereich von 30 bis 70 % und ganz besonders bevorzugt aus einem Bereich von 30 bis 50 % eingesetzt. Die Herstellung derartiger Metalleffektpigmente sind in der WO 2004/087816 A2 oder der WO 2008/077612 A2 beschrieben, die hiermit unter Bezugnahme aufgenommen sind.

Bei einer weiteren Ausführungsform beträgt die relative Standardabweichung der Dickenverteilung der metallischen plättchenförmigen Substrate 11% bis 98%, bevorzugt 22% bis 78%, besonders bevorzugt 28% bis 68% und ganz besonders bevorzugt 34% bis 64%. Die relative Standardabweichung in [%] ist dabei der Quotient aus berechneter Standardabweichung der Dickenverteilung und mittlerer Dicke $h_{50}$.

Die erfindungsgemäßen, bevorzugt in einer Nagellackkomposition zu verwendenden, oberflächenmodifizierten Effektpigmente basierend auf metallischen, nicht beschichteten, plättchenförmigen Substraten können optional wenigs-

tens eine Schicht aus oder mit wenigstens einem Metalloxid, Metallhydroxid und/oder Metalloxidhydrat aufweisen, wobei das Metallion ausgewählt aus der Gruppe der Metalle, bestehend aus Al, Si, Sn, Zn Ti und Fe, bevorzugt ausgewählt aus der Gruppe de Metalle, bestehend aus Al und Si, ist. Vorstehend aufgeführte Metalloxide, Metallhydroxide und/oder Metalloxidhydrate können als (i) separate Schichten, (ii) gemischtes Metalloxid, gemischtes Metallhydroxid und/oder gemischtes Metalloxidhydrat oder (iii) in separaten Schichten auf dem metallischen plättchenförmigen Substrat vorliegen.

Die erfindungsgemäßen, bevorzugt in einer Nagellackkomposition zu verwendenden, oberflächenmodifizierten Effektpigmente basierend auf metallischen plättchenförmigen Substraten können eine beliebige mittlere Teilchengröße $D_{50}$ aufweisen. Die $D_{50}$-Werte der erfindungsgemäßen, bevorzugt in einer Nagellackkomposition zu verwendenden, oberflächenmodifizierten Effektpigmente basierend auf metallischen plättchenförmigen Substraten liegen bevorzugt in einem Bereich von 2 µm bis 150 µm, weiter bevorzugt in einem Bereich von 2,5 µm bis 170 µm, weiter bevorzugt in einem Bereich von 3 µm bis 140 µm, besonders bevorzugt in einem Bereich von 3,5 µm bis 90 µm und ganz besonders bevorzugt in einem Bereich von 3,8 µm bis 56 µm. Äußerst bevorzugt weisen die erfindungsgemäßen, bevorzugt in einer Nagellackkomposition zu verwendenden, oberflächenmodifizierten Effektpigmente basierend auf metallischen plättchenförmigen Substraten einen $D_{50}$-Wert aus einem Bereich 2,5 µm bis 14 µm oder aus einem Bereich von 15 µm bis 35 µm auf. Unter 2 µm nehmen die Glanzwerte deutlich ab und oberhalb von 150 µm erhält man keine einheitlich geschlossene metallische Oberfläche.

Die $D_{10}$-Werte der erfindungsgemäßen, bevorzugt in einer Nagellackkomposition zu verwendenden, oberflächenmodifizierten Effektpigmente basierend auf metallischen plättchenförmigen Substraten liegen bevorzugt in einem Bereich von 1 µm bis 60 µm, weiter bevorzugt in einem Bereich von 2 µm bis 40 µm, besonders bevorzugt in einem Bereich von 4 µm bis 31 µm und ganz besonders bevorzugt in einem Bereich von 5 µm bis 19 µm. Unter 1 µm nehmen die Glanzwerte deutlich ab und oberhalb von 60 µm erhält man keine einheitlich geschlossene metallische Oberfläche.

Die $D_{90}$-Werte der erfindungsgemäßen, bevorzugt in einer Nagellackkomposition zu verwendenden, oberflächenmodifizierten Effektpigmente basierend auf metallischen plättchenförmigen Substraten liegen bevorzugt in einem Bereich von 10 µm bis 600 µm, weiter bevorzugt in einem Bereich von 30 µm bis 200 µm, besonders bevorzugt in einem Bereich von 40 µm bis 150 µm und ganz besonders bevorzugt in einem Bereich von 45 µm bis 120 µm. Unter 10 µm nehmen die Glanzwerte deutlich ab und oberhalb von 600 µm erhält man keine einheitlich geschlossene metallische Oberfläche.

Bei einer Ausführungsform besitzen die erfindungsgemäßen, bevorzugt in einer Nagellackkomposition zu verwendenden, oberflächenmodifizierten Effektpigmente basierend auf metallischen plättchenförmigen Substraten einen Span ΔD, definiert als $\Delta D = \frac{D_{90} - D_{10}}{D_{50}}$, aus einem Bereich von 0,7 bis 2,5, bevorzugt aus einem Bereich von 0,8 bis 2,2, weiter bevorzugt aus einem Bereich von 0,9 bis 1,9, besonders bevorzugt aus einem Bereich von 0,9 bis 1,8 und ganz besonders bevorzugt aus einem Bereich von 1 bis 1,7.

Bei einer weiteren Ausführungsform weisen die erfindungsgemäßen, bevorzugt in einer Nagellackkomposition zu verwendenden, oberflächenmodifizierten Effektpigmente basierend auf metallischen plättchenförmigen Substraten ein Aspektverhältnis, definiert als Quotient aus $D_{50}$ und mittlerer Dicke, bevorzugt aus einem Bereich von 10 bis 2000, weiter bevorzugt aus einem Bereich von 30 bis 1200, besonders bevorzugt aus einem Bereich von 100 bis 1100 und ganz besonders bevorzugt aus einem Bereich von 200 bis 1000 auf. Unterhalb der Untergrenzen sind die optischen Eigenschaften der metallischen Effektpigmente noch nicht ausreichend zur Erreichung eines Spiegelglanzes und oberhalb von 2000 sind die metallischen Effektpigmente nur sehr schwer herstellbar.

Bei einer weiteren Ausführungsform weisen die erfindungsgemäßen, bevorzugt in einer Nagellackkomposition zu verwendenden, oberflächenmodifizierten Effektpigmente basierend auf metallischen plättchenförmigen Substraten bevorzugt eine mittlere Gesamtdicke $h_{50}$ aus einem Bereich von 20 nm bis 4000 nm, weiter bevorzugt aus einem Bereich von 30 nm bis 3000 nm, besonders bevorzugt aus einem Bereich von 70 nm bis 2000 nm und ganz besonders bevorzugt aus einem Bereich von 230 nm bis 1300 nm auf. Unter mittlerer Gesamtdicke wird die komplette mittlere Dicke des oberflächenmodifzierten Effektpigments, also metallisches plättchenförmiges Substrat plus optionale Beschichtung plus Oberflächenmodifizierung, verstanden.

Erfindungsgemäß umfassen die bevorzugt in einer Nagellackkomposition zu verwendenden, oberflächenmodifizierten Effektpigmente als metallisches plättchenförmiges Substrat Metallplättchen hergestellt durch ein PVD-Verfahren mit einem $D_{50}$-Wert aus einem Bereich von 2,5 µm bis 90 µm, weiter bevorzugt aus einem Bereich von 8 µm bis 25 µm und einer mittleren Dicke aus einem Bereich von 13 nm bis 60 nm eingesetzt. Bei dieser Ausführungsform werden die Aluminiumplättchen vorzugsweise nicht beschichtetet. Die auf Aluminiumplättchen vorhandene natürliche Oxidschicht wird hierbei nicht als aufgebrachte Beschichtung verstanden.

Überraschenderweise können auch Spiegelglanzeffekte mit sehr kleinen PVD-Pigmenten erreicht werden. Diese Pigmente können z.B. hergestellt werden, indem man gewöhnliche PVD-Pigmente weiter vermahlt oder durch Ultraschalleinwirkung zerkleinert. Erfindungsgemäß handelt es sich bei diesen Metallplättchen um Aluminiumplättchen. Besonders bevorzugt weisen diese Aluminium-PVD Pigmente einen $D_{50}$-Wert aus einem Bereich von 6 µm bis 18 µm und einer mittleren Dicke aus einem Bereich von 14 nm bis 40 nm auf. Ganz besonders bevorzugt sind Aluminium-PVD

Pigmente mit einen $D_{50}$-Wert aus einem Bereich von 7 µm bis 16 µm und einer mittleren Dicke aus einem Bereich von 15 nm bis 35 nm.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der vermessenen oberflächenmodifizierten Effektpigmente einen volumengemittelten Durchmesser aufweisen, der gleich oder kleiner als der jeweils angegebene Wert ist. Hierbei wird die Größenverteilungskurve der erfindungsgemäßen, bevorzugt in einer Nagellackkomposition zu verwendenden, oberflächenmodifizierten Effektpigmente sowie der oberflächenmodifizierten Effektpigmente gemäß Anspruch 1, jeweils basierend auf nichtmetallischen plättchenförmigen Substraten, mit dem Gerät Mastersizer 2000 der Fa. Malvern gemäß Herstellerangaben bestimmt. Die Auswertung der Streulichtsignale erfolgt nach der Fraunhofer-Theorie, welche auch Brechungs- und Absorptionsverhalten der Partikel beinhaltet. Die Größenverteilungskurve der erfindungsgemäßen, bevorzugt in einer Nagellackkomposition zu verwendenden, oberflächenmodifizierten Effektpigmente sowie der oberflächenmodifizierten Effektpigmente gemäß Anspruch 1, jeweils basierend auf metallischen plättchenförmigen Substraten, wird mit dem Gerät Cilas 1064 der Fa. Quantachrome oder dem Gerät Horiba LA-930 der Fa. Horiba ermittelt. Die Auswertung der Streulichtsignale erfolgt nach der Fraunhofer-Theorie, welche auch Brechungs- und Absorptionsverhalten der Partikel beinhaltet.

Es hat sich gezeigt, dass die erfindungsgemäß oberflächenmodifizierten Effektpigmente nicht in allen Nagellacksystemen, wie sie zum Beispiel in der EP 1796794 B2 offenbart wurden, gute Ergebnisse zeigt. Häufig geht der Leafing-Effekt bei der Applikation oder danach verloren und damit einhergehend kommen die optischen Eigenschaften der Effektpigmente nicht vollkommen zur Geltung.

Daher ist ein wesentlicher Bestandteil der vorliegenden Erfindung die Bereitstellung eines Nagellacks, in dem die leafing-Eigenschaften der oberflächenmodifizierten Effektpigmente sehr gut zur Geltung kommen.

Die Erfindung richtet sich daher auch auf eine Nagellackkomposition enthaltend

a) wenigstens ein mit einem Ausgangsmaterial (Additiv) oberflächenmodifiziertes Effektpigment wobei das Effektpigment ein plättchenförmiges Substrat und optional wenigstens eine auf dem Substrat aufgebrachte Beschichtung umfasst

b) wenigstens ein kohlenwasserstoffhaltiges Harz und

c) wenigstens ein Lösemittel oder Lösemittelgemisch,

wobei als Ausgangsmaterial (Additiv) zur Oberflächenmodifizierung des Effektpigments wenigstens eine Verbindung aus der Gruppe bestehend aus phosphorsäureesterhaltigen, phosphonsäureesterhaltigen, phosphonsäurehaltigen, fettsäurehaltigen und/oder silanhaltigen Verbindungen oder Mischungen hiervon, entnommen wird.

Bei den zur Oberflächenmodifizierung einsetzbaren Phosphorsäureestern und/oder Phosphorsäuren kann es sich um Phosphorsäureester der allgemeinen Formel $(R\text{-}O)_n\text{-}P(O)(OR')(OR'')_m$ handeln, wobei die Reste R, R' und R" bevorzugt folgende Bedeutung haben:

R = linearer und/oder verzweigter Alkylrest mit einer Kohlenstoffkette aus einem Bereich von $C_{10}$ bis $C_{20}$ und R' = R" = H, linearer und/oder verzweigter Alkylrest mit einer Kohlenstoffkette aus einem Bereich von $C_1$ bis $C_6$, bevorzugt $C_1$ bis $C_3$, wobei R' und

R" identisch oder verschieden voneinander sein können und wobei n = 1 oder 2 und m = n - 1 und n + m = 2 ist. Hierbei können hinsichtlich der Zahl n sowohl reine Formen der Monoester (n = 1) oder Diester (n = 2) als auch Mischungen hiervon vorliegen.

Bei einer besonders bevorzugten Ausführungsform werden zur Oberflächenmodifzierung der in einer erfindungsgemäßen Nagellackkomposition einzusetzenden Effektpigmente primäre Phosphorsäuren oder Phosphonsäuren mit R = linearer, unsubstituierter Alkylrest mit einer Kohlenstoffkette aus einem Bereich von $C_{12}$ bis $C_{18}$, vorzugsweise aus einem Bereich von $C_{14}$ bis $C_{18}$ und R' = R" = H eingesetzt. Weiter bevorzugt ist hierbei n = 1 (Monoester).

Eine bevorzugte Phosphonsäure ist Laurylphosphonsäure und bevorzugte Phosphorsäuren sind Cetylphosphat oder Stearylphosphat.

Zur Oberflächenmodifizierung können beispielsweise 2-Ethylhexylphosphorsäureester (CAS: 12645-31-7), Laurylphosphorsäureester (CAS: 12751-23-4), Cetylphosphorsäureester (CAS: 3539-43-3), Stearylphosphorsäureester (CAS: 39471-52-8) und/oder Monoethylmono-(9Z)-9-octadecenylphosphorsäureester (CAS: 10483-96-2) eingesetzt werden.

Besonders bevorzugt sind Cetylphosphorsäureester, Stearylphosphorsäureester oder Laurylphosphorsäureester und besonders bevorzugt sind Cetylphosphorsäureester oder Stearylphosphorsäureester und ganz besonders bevorzugt sind Cetylphosphorsäureester.

Zur Herstellung der erfindungsgemäßen, bevorzugt in einer Nagellackkomposition zu verwendenden, oberflächenmodifizierten Effektpigmente wird das Ausgangsmaterial (Additiv) zur Oberflächenmodifizierung bevorzugt in

einer Menge aus einem Bereich von 1,5 Gew.-% bis 50 Gew.-%, besonders bevorzugt aus einem Bereich von 3,2 Gew.-% bis 40 Gew.-% und ganz besonders bevorzugt aus einem Bereich von 4,8 Gew.-% bis 23 Gew.-%, jeweils bezogen auf das Gesamtgewicht des eingesetzten Effektpigments, eingesetzt.

Da sich die hier angegebenen Mengen des Additivs auf das Ausgangsmaterial beziehen, kann die tatsächliche Additivmenge des fertig beschichteten Effektpigments geringer sein, da z.B. bei einer Menge von 50 Gew.-% nicht alles Additiv auf die Pigmentoberfläche aufziehen kann. Dementsprechend können auch geringere Mengen der leafing-Additive in den die Pigmente enthaltenden Anwendungen, insbesondere in Nagellacken gefunden werden.

Die recht hohen Additivmengen im Ausgangsmaterial bewirken eine sehr hohe und dichte Beschichtung der Effektpigmentoberfläche mit dem Additiv.

Bei ganz besonders bevorzugten Ausführungsformen werden wenigstens ein Phosphorsäureester und/oder wenigstens eine Phosphorsäure der Formel $(R\text{-}O)_n\text{-}P(O)(OR')(OR'')_m$, wobei jeweils R = linearer Alkylrest mit einer Kohlenstoffkette aus einem Bereich von $C_{14}$ bis $C_{18}$, und wobei R' und R" unabhängig voneinander H, $CH_3$, $C_2H_5$ oder $C_3H_8$ sind und wobei n = 1 oder 2 und m = n - 1 und n + m = 2 ist, in einer Gesamtmenge aus einem Bereich von 14 Gew.-% bis 40 Gew.-%, bezogen auf das Gesamtgewicht des eingesetzten Effektpigments, als Ausgangsmaterial (Additiv) zur Oberflächenmodifizierung von Effektpigmenten basierend auf metallischen plättchenförmigen Substraten, die durch PVD-Verfahren hergestellt wurden mit einer mittleren Substratdicke $h_{50}$ aus einem Bereich von 15 nm bis 40 nm, bevorzugt 20 bis 40 nm verwendet. Weiter bevorzugt ist hierbei R' = R" = H. Zudem ist besonders bevorzugt, dass n = 1 ist.

Unter "Gesamtmenge" wird erfindungsgemäß die komplette Menge an Ausgangsmaterial (Additiv) verstanden und zwar unabhängig davon, ob es sich ausschließlich um wenigstens einen Phosphorsäureester oder ausschließlich um wenigstens einen Phosphonsäureester oder um eine Mischung aus wenigstens einem Phosphorsäureester und wenigstens einem Phosphonsäureester oder um eine Mischung unterschiedlicher Phosphonsäuren oder um eine Mischung unterschiedlicher Fettsäuren oder um eine Mischung unterschiedlicher Silane handelt.

Die erfindungsgemäßen oberflächenmodifizierten Effektpigmente gemäß Anspruch 1 können in kosmetischen Formulierungen, insbesondere in Nagellackkompositionen, Verwendung finden. Die oberflächenmodifizierten Effektpigmente gemäß Anspruch 1 zeichnen sich insbesondere in Nagellackkompositionen durch ihr ausgezeichnetes Leafing-Verhalten aus.

**Erfindungsgemäße Nagellackkomposition:**

Die Erfindung richtet sich weiterhin auf eine Nagellackkomposition, die den leafing-Effekt der Effektpigmente in ausgezeichneter Weise ermöglicht und erhält.

Diese erfindungsgemäße Nagellackkomposition enthält:

a) wenigstens ein mit einem Ausgangsmaterial (Additiv) oberflächenmodifiziertes Effektpigment wobei das Effektpigment ein plättchenförmiges Substrat und optional wenigstens eine auf dem Substrat aufgebrachte Beschichtung umfasst
b) wenigstens ein Kohlenwasserstoffharz als Bindemittel und
c) wenigstens ein Lösemittel oder Lösemittelgemisch,

wobei als Ausgangsmaterial (Additiv) zur Oberflächenmodifizierung des Effektpigments wenigstens eine Verbindung aus der Gruppe bestehend aus phosphorsäureesterhaltigen, phosphonsäureesterhaltigen, phosphonsäurehaltigen, fettsäurehaltigen und/oder silanhaltigen Verbindungen oder Mischungen hiervon, entnommen wird.

Die erfindungsgemäße Nagellackkomposition, welche wenigstens ein oberflächenmodifiziertes Effektpigment umfasst, weist im Unterschied zu den meisten kommerziell erhältlichen Nagellackkompositionen vorzugsweise keine Nitrocellulose auf. Das optische Erscheinungsbild der erfindungsgemäßen Nagellackkomposition wird, nach Applikation und Trocknung, maßgeblich durch das wenigstens eine oberflächenmodifizierte Effektpigment bestimmt.

Die erfindungsgemäße Nagellackkomposition umfasst das wenigstens eine oberflächenmodifizierte Effektpigment bevorzugt in einem Anteil aus einem Bereich von 0,3 Gew.-% bis 8,5 Gew.-%, weiter bevorzugt aus einem Bereich von 0,5 Gew.-% bis 5,0 Gew.-%, besonders bevorzugt aus einem Bereich von 0,6 Gew.-% bis 3,0 Gew.-% und ganz besonders bevorzugt aus einem Bereich von 0,8 Gew.-% bis 1,9 Gew.-%, jeweils bezogen auf das Gesamtgewicht der Nagellackkomposition.

Bei einer besonders bevorzugten Ausführungsform umfasst die erfindungsgemäße Nagellackkomposition wenigstens ein oberflächenmodifiziertes, optional beschichtetes, Aluminiumpigment, wobei das oberflächenmodifizierte Aluminiumpigment einen $D_{50}$-Wert aus einem Bereich von 5 $\mu$m bis 100 $\mu$m, bevorzugt aus einem Bereich von 2 $\mu$m bis 60 $\mu$m, aufweist und die mittlere Dicke des Aluminiumplättchens in einem Bereich von 10 nm bis 100 nm, bevorzugt in einem Bereich von 20 nm bis 40 nm, liegt. Bei dieser Ausführungsform wird als Ausgangsmaterial (Additiv) zur Oberflächenmodifizierung des optional beschichteten Aluminiumpigments bevorzugt wenigstens ein Phosphorsäureester R-O-P(O)(OR')(OR") mit R = linearer Alkylrest mit einer Kohlenstoffkette aus einem Bereich von $C_{14}$ bis $C_{18}$, R' = R" = H in einem

Anteil aus einem Bereich von 10 Gew.-% bis 50 Gew.-%, bezogen auf das Gesamtgewicht des eingesetzten Effektpigments, eingesetzt. Die erfindungsgemäße Nagellackkomposition umfasst hierbei das vorstehend beschriebene oberflächenmodifizierte, optional beschichtete, Aluminiumpigment bevorzugt in einem Anteil aus einem Bereich von 0,6 Gew.-% bis 3,0 Gew.-%, besonders bevorzugt aus einem Bereich von 0,8 Gew.-% bis 1,9 Gew.-%, jeweils bezogen auf das Gesamtgewicht der Nagellackkomposition.

Unterhalb der bei den erfindungsgemäß oberflächenmodifizierten Effektpigmenten gemäß Anspruch 1 bzw. der verschiedenen, vorstehend beschriebenen Effektpigment-/Additivkombinationen für die jeweils für verschiedene Effektpigmenttypen angegebenen Mengen der als Ausgansmaterial (Additiv) zur Oberflächenmodifizierung zu verwendenden spezifischen Substanzen findet kein ausreichendes leafing der Effektpigmente statt. Oberhalb der jeweils angegebenen Mengen der Additive können gegebenenfalls zu große Mengen der Additive in die fertige Nagellackkomposition eingetragen werden.

Hierbei ist es besonders bevorzugt, dass die Effektpigmente in einem separaten Schritt mit dem Additiv beschichtet werden, bevor sie in das Nagellacksystem eingetragen werden.

Ein bevorzugtes Verfahren zur Herstellung der erfindungsgemäßer oberflächenmodifizierten Effektpigmente umfasst daher die folgenden Schritte:

i. Suspendieren des optional mit wenigstens einem Metalloxid, Metallhydroxid und/oder Metalloxidhydrat beschichteten, metallischen plättchenförmigen Substrats oder des optional mit wenigstens einem Metalloxid, Metallhydroxid und/oder Metalloxidhydrat beschichteten nichtmetallischen plättchenförmigen Substrats in wenigstens einem Lösemittel,
ii. Zugabe des Phosphorsäurecetylesters oder des Phosphorsäurestearylesters bei optional erhöhter Temperatur zu der Suspension aus Schritt i. und Rühren der nun erhaltenen Suspension,
iii. Filtrieren, optional Trocknen, des gemäß Schritt ii. erhaltenen oberflächenmodifizierten Effektpigments.

Als Lösemittel wird bevorzugt ein mit dem erfindungsgemäßen Nagellack kompatibles Lösemittel verwendet. Besonders bevorzugt wird Butylacetat verwendet.

Bei PVD-Metallpigmenten oder bei besonders dünnen, durch Nassvermahlung hergestellten Metallpigmente wird man auf den Trocknungsschritt nach iii. verzichten und statt dessen diese Pigmente in einer Dispersion belassen.

Ein Teil der eingesetzten Additive wird nicht auf der Oberfläche des Effektpigments anhaften, da die Oberfläche bereits abgesättigt ist. Für eine hohe und gleichmäßige Bedeckung ist es jedoch wichtig, ausreichende Mengen an Additiv zur Verfügung zu stellen, da nur dann ein starker leafing-Effekt zu erwarten ist.

In den erfindungsgemäßen Nagellackkompositionen ordnen sich die oberflächenmodifizierten Effektpigmente bevorzugt an der Oberfläche des Klarlacks, also der erfindungsgemäßen Nagellackkomposition ohne oberflächenmodifizierte Effektpigmente, an. Unter "an der Oberfläche anordnen" wird erfindungsgemäß verstanden, dass sich die oberflächenmodifizierten Effektpigmente auf der Nagellackbasis und/oder ausgehend von der Grenzfläche Nagellackkomposition/Luft bzw. Nagellackkomposition/Überlackierung in Richtung des lackierten Untergrunds im an diese Grenzfläche anschließenden Drittel der Nagellackkomposition befinden. Bevorzugt schwimmen die oberflächenmodifizierten Effektpigmente im Klarlack auf und richten sich an der Klarlackoberfläche aus. Die oberflächenmodifizierten Effektpigmente zeichnen sich mithin in der erfindungsgemäßen Nagellackkomposition durch ein ausgeprägtes leafing-Verhalten aus.

Aufgrund dieses ausgeprägten leafing-Verhaltens der oberflächenmodifizierten Effektpigmente lassen sich erfindungsgemäß Nagellackkompositionen herstellen, welche ihr optisches Erscheinungsbild hauptsächlich dem der Nagellackbasis zugesetzten wenigstens einem oberflächenmodifizierten Effektpigment verdanken. In Abhängigkeit von dem wenigstens einem oberflächenmodifizierten Effektpigment sind so auf einfache Art und Weise Nagellackkompositionen zugänglich, welche sich nach Applikation und Trocknung durch ihren metallischen Charakter, ihren perlmuttartigen Schimmer, ihre Interferenzfarbe, einen Farbwechsel bei unterschiedlichem Betrachtungswinkel, intensive Glitzereffekte und/oder seidenmattes Aussehen auszeichnen, um nur einige erzielbare Effekte beispielhaft zu nennen. Selbstverständlich können der Nagellackkomposition je nach zu erzielendem optischen Effekt auch verschiedene oberflächenmodifizierte Effektpigmente zugesetzt werden, wobei hierbei sowohl die Oberflächenmodifizierung als auch die Effektpigmente verschieden voneinander sein können. Unter "voneinander verschiedenen Effektpigmenten" werden auch in ihrer Art identische Effektpigmente, beispielsweise Aluminiumpigmente, verstanden, die sich jedoch beispielsweise in ihrer Teilchengröße voneinander unterscheiden.

Als eine Besonderheit bei der Verwendung oberflächenmodifizierter Effektpigmente basierend auf Aluminiumplättchen oder basierend auf mit metallischem Silber beschichteten nichtmetallischen plättchenförmigen Substraten sei noch erwähnt, dass sich hiermit, nach Applikation und Trocknung Nagellackkompositionen mit Spiegelglanz erhalten lassen. Im Idealfall weisen die erfindungsgemäßen Nagellackkompositionen dann nach Applikation und Trocknung eine derart hohe Abbildschärfe auf, so dass ein Betrachter sich darin nahezu spiegeln kann.

Unter "Spiegelglanz" wird erfindungsgemäß verstanden, dass die Nagellackkomposition nach Applikation auf

Glasplatten mittels einer Kastenrakel (Filmapplicator Erichsen System Wasag Model 288, Fa. Erichsen) in einer Nassfilmschichtdicke von 110 $\mu$m und nach anschließender Trocknung bei Raumtemperatur wenigstens 120 Glanzeinheiten, gemessen bei der 20° Geometrie (Byk-Gardner, Digitaler Katalog "Qualitätskontrolle für Lacke und Kunststoffe", Seite 16) und wenigstens 1100 Hazeeinheiten (Hlog) aufweist. Unter einem ausgezeichneten Spiegelglanz wird erfindungsgemäß verstanden, wenn wenigstens 150 Glanzeinheiten, gemessen bei der 20° Geometrie (Byk-Gardner, Digitaler Katalog "Qualitätskontrolle für Lacke und Kunststoffe", Seite 16) und wenigstens 1100 Hazeeinheiten (Hlog) erreicht werden. Die Glanz- und Hazeeinheiten (Hlog) werden hierbei vorzugsweise mit dem Messgerät haze-gloss, Fa. Byk-Gardner, bestimmt. Weisen die erfindungsgemäßen Nagellackapplikationen mikroskopisch kleine Strukturen auf, so wird hieran Licht nahe dem Reflexionswinkel gestreut, was die Abbildeschärfe der Nagellackapplikationen reduziert. Glanz (20° Geometrie) und Haze werden bei der Beurteilung des Spiegelglanzes vorzugsweise nicht unabhängig voneinander betrachtet. Je höher der Zahlenwert der Glanzeinheiten bei der 20° Geometrie ist, desto niedriger kann der Zahlenwert der Hazeeinheiten (Hlog) sein bzw. umgekehrt, je höher der Zahlenwert der Hazeeinheiten (Hlog) ist, desto niedriger kann der Zahlenwert der Glanzeinheiten bei der 20° Geometrie sein, um vom menschlichen Auge noch als Spiegelglanz wahrgenommen zu werden. Je höher der Zahlenwert der Glanzeinheiten bei der 20° Geometrie und je höher der Zahlenwert der Hazeeinheiten (Hlog) ist, desto höher ist die visuell wahrgenommene Abbildeschärfe der Nagellackapplikation.

Bei einer ganz bevorzugten Ausführungsform weisen die erfindungsgemäßen Nagellackkompositionen nach Applikation auf Glasplatten mittels einer Kastenrakel (Filmapplicator Erichsen System Wasag Model 288, Fa. Erichsen) in einer Nassfilmschichtdicke von 110 $\mu$m und nach anschließender Trocknung bei Raumtemperatur wenigstens 200 Glanzeinheiten, bevorzugt wenigstens 300 Glanzeinheiten, besonders bevorzugt wenigstens 400 Glanzeinheiten, ganz besonders bevorzugt wenigstens 450 bis 1500 Glanzeinheiten, jeweils gemessen bei der 20° Geometrie, und wenigstens 1200 Hazeeinheiten (Hlog), bevorzugt wenigstens 1300 Hazeeinheiten (Hlog), besonders bevorzugt wenigstens 1400 Hazeeinheiten (Hlog) und ganz besonders bevorzugt wenigstens 1410 bis 2000 Hazeeinheiten (Hlog) auf. Auch bei dieser Ausführungsform ist bei einem niedrigeren Zahlenwert der Glanzeinheiten, gemessen bei der 20° Geometrie, ein höherer Zahlenwert der Hazeeinheiten (Hlog) bevorzugt.

Bei einer bevorzugten Ausführungsform umfassen die erfindungsgemäßen Nagellackkompositionen, welche sich nach Applikation und Trocknung durch einen ausgezeichneten Spiegelglanz auszeichnen, 0,4 Gew.-% bis 2,7 Gew.-%, bevorzugt 0,5 Gew.-% bis 1,8 Gew.-%, jeweils bezogen auf das Gesamtgewicht der Nagellackkomposition, oberflächenmodifizierte Effektpigmente basierend auf Aluminiumplättchen, wobei die Aluminiumplättchen einen $D_{50}$-Wert aus einem Bereich von 2 $\mu$m bis 500 $\mu$m, bevorzugt aus einem Bereich von 2,5 $\mu$m bis 90 $\mu$m, und eine mittlere Dicke aus einem Bereich von 15 nm bis 1000 nm, bevorzugt aus einem Bereich von 18 nm bis 60 nm, aufweisen.

Bei einer besonders bevorzugten Ausführungsform umfassen die erfindungsgemäßen Nagellackkompositionen, welche sich nach Applikation durch einen ausgezeichneten Spiegelglanz auszeichnen, 0,6 Gew.-% bis 4,9 Gew.-%, bevorzugt 0,3 Gew.-% bis 3,8 Gew.-%, jeweils bezogen auf das Gesamtgewicht der Nagellackkomposition, oberflächenmodifizierte Effektpigmente basierend auf Aluminiumplättchen, wobei die Aluminiumplättchen einen $D_{50}$-Wert aus einem Bereich von 5 $\mu$m bis 150 $\mu$m, bevorzugt aus einem Bereich von 10 $\mu$m bis 60 $\mu$m, und eine mittlere Dicke aus einem Bereich von 10 nm bis 600 nm, bevorzugt aus einem Bereich von 20 nm bis 100 nm, aufweisen.

Bei einer bevorzugten Ausführungsform weisen die erfindungsgemäßen Nagellackkompositionen nach Applikation auf einer Glasplatte mittels einer Kastenrakel (Filmapplicator Erichsen System Wasag Model 288, Fa. Erichsen) in einer Nassfilmschichtdicke von 100 $\mu$m und anschließender Trocknung bei Raumtemperatur wenigstens 200 Glanzeinheiten, gemessen bei der 20° Geometrie, und wenigstens 1200 Hazeeinheiten (Hlog) auf.

Bei einer bevorzugten Ausführungsform umfasst die erfindungsgemäße Nagellackkomposition wenigstens ein oberflächenmodifiziertes Effektpigment in einem

Anteil aus einem Bereich von 0,1 Gew.-% bis 8,4 Gew.-%, bevorzugt in einem Anteil aus einem Bereich von 0,15 Gew.-% bis 6,9 Gew.-%, besonders bevorzugt in einem Anteil aus einem Bereich von 0,2 Gew.-% bis 4,3 Gew.-%, jeweils bezogen auf das Gesamtgewicht der Nagellackkomposition, wenigstens ein kohlenwasserstoffhaltiges Harz und wenigstens ein Lösemittel.

Bei einer weiteren bevorzugten Ausführungsform umfasst die erfindungsgemäße Nagellackkomposition wenigstens ein oberflächenmodifiziertes Effektpigment basierend auf einem metallischen plättchenförmigen Substrat, wobei das Effektpigment eine mittlere Teilchengröße $D_{50}$ aus einem Bereich von 2 $\mu$m bis 60 $\mu$m und eine mittlere Gesamtdicke aus einem Bereich von 10 nm bis 150 nm aufweist.

Bindemittel:

Die erfindungsgemäßen Nagellackkompositionen umfassen als Bindemittel wenigstens ein Kohlenwasserstoffharz, wobei das Bindemittel bevorzugt einen Bindemittelfestkörperanteil aus einem Bereich von 25 Gew.-% bis 64 Gew.-%, weiter bevorzugt aus einem Bereich von 25 Gew.-% bis 60 Gew.-%, weiter bevorzugt aus einem Bereich von 28 Gew.-% bis 55 Gew.-%, besonders bevorzugt aus einem Bereich von 29 Gew.-% bis 50 Gew.-% und ganz besonders bevorzugt

aus einem Bereich von 35 Gew.-% bis 43 Gew.-%, jeweils bezogen auf das Gesamtgewicht der Nagellackkomposition, aufweist.

Unterhalb eines Bindemittelanteils von 25 Gew.-% waren keine guten optischen Effekte durch die Effektpigmente im applizierten Nagellack mehr zu erkennen.

Oberhalb von 60 Gew.-% lässt die optische Qualität der Effektpigmente ebenfalls nach und zunehmend wird die Viskosität der erfindungsgemäßen Nagellackkompositionen zu hoch.

Die erfindungsgemäßen Nagellackkompositionen umfassen vorzugsweise als Bindemittel Kohlenwasserstoffharze mit einem mittleren Molekulargewicht ($M_w$) aus einem Bereich von 800 bis 6000, bevorzugt aus einem Bereich 900 bis 5000, oder aus einem Bereich von 8000 bis 10000, bevorzugt aus einem Bereich von 8500 bis 9300. Das mittlere Molekulargewicht $M_W$ wurde mittels Gel-Permeations-Chromatographie (GPC) mit einem Polystyrol Standard bestimmt.

Unter Kohlenwasserstoffharze versteht man synthetische Harze, die durch Reaktion von Kohlenwasserstoffen (außer Olefinen) mit sich selbst in Gegenwart von Aluminiumchlorid oder Schwefelsäure als Katalysator entstehen. Die Kohlenwasserstoffharze werden entsprechend ihres Aufbaues in Petrolharze, Terpenharze und Cumaron-Inden-Harze unterteilt. Zu den Kohlenwasserstoffharze werden auch die Reaktionsprodukte aus Xylol und Formaldehyd, die Xylol-Formaldehyd-Harze gerechnet.

Die Herstellung der Kohlenwasserstoffharze erfolgt in bekannter Weise durch Erhitzen von hochsiedenden Fraktionen der Benzinpyrolyse (Pyrolyseöl) oder der isoprenfreien $C_5$-Fraktion der Benzinpyrolyse in Anwesenheit von Aluminiumchlorid. Die Kohlenwasserstoffharze sind in den meisten organischen Lösungsmitteln, z. B. Ester, Ether, Chlorkohlenwasserstoffe und Aromaten, löslich.

Ohne an eine Theorie gebunden zu sein, vermuten die Erfinder, dass bei der Verwendung von polaren Bindemitteln die mit geeigneten Additiven beschichteten Effektpigmente immer noch teilweise von dem Bindemittel benetzt werden und daher nicht den gewünschten leafing- Effekt aufweisen. Hingegen sind die Harze der erfindungsgemäßen Nagellackkomposition sehr unpolar und benetzen demzufolge die Effektpigmente nicht. Dadurch können die Effektpigmente den leafing-Effekt besser ausbilden.

In bevorzugten Ausführungsformen werden Nagellackkompositionen enthaltend aromatische Kohlenwasserstoffharze verwendet.

Die erfindungsgemäßen Nagellackkompositionen können als Bindemittel Kohlenwasserstoffharze, wie beispielsweise Kristalex F100 Hydrocarbon Resin, Kristalex 5140 Hydrocarbon Resin, Kristalex 3070 Hydrocarbon Resin, Kristalex 3085 Hydrocarbon Resin, Kristalex F115 Hydrocarbon Resin, jeweils Fa. Eastman umfassen.

Bevorzugt umfassen die erfindungsgemäßen Nagellackkompositionen als Bindemittel die Kohlenwasserstoffharze Kristalex F100 Hydrocarbon Resin und Kristalex 5140 Hydrocarbon Resin.

In besonders bevorzugten Ausführungsformen enthält die erfindungsgemäße Nagellackkomposition kohlenwasserstoffhaltiges Harz in einer Menge, die 80 bis 100 Gew.-%, weiter bevorzugt 90 bis 99 Gew.-% des gesamten organischen Bindemittels ausmacht.

Die Verwendung von Kohlenwasserstoffharzen in Nagellacken als Hauptbestandteil des Bindemittels ist nach den Kenntnissen der Erfinder nicht üblich. Gewöhnlicherweise sind Kohlenwasserstoffharze sehr selten Bestandteile von Nagellacken und wenn, so werden sie mit anderen Bindemitteln in vergleichsweise eher geringen Anteilen eingesetzt.

Bei einer besonders bevorzugten Ausführungsform umfassen die erfindungsgemäßen Nagellackkompositionen wenigstens zwei voneinander verschiedene Kohlenwasserstoffharze mit einem ersten mittleren Molekulargewicht $M_W$ von 1200 bis 1600 g/mol und einem zweiten mittleren Molekulargewicht $M_W$ 4500 bis 5500 g/mol im Gewichtsverhältnis 1:1 bis 1:10, bevorzugt 1:1 bis 1:8, besonders bevorzugt 1:1 bis 1:4 und ganz besonders bevorzugt 1:1 bis 1:2 der beiden verschiedenen Kohlenwasserstoffharze.

In weitere Ausführungsformen enthält die Nagellackkomposition keine oder nahezu keine zusätzlichen Bindemittel der Gruppe aus Nitrocellulose, Polyesterharze, Polyvinylharze, Alkydharz, Epoxidharze oder Celluloseacetatbutyrat. Diese Bindemittel sind bevorzugt in Mengenanteilen von unter 10 Gew.-%, weiter bevorzugt unter 5 Gew.-% und besonders bevorzugt von unter 1 Gew.-% und ganz besonders bevorzugt unter 0,1 Gew.-%, jeweils bezogen auf das Gesamtgewicht der Kohlenwasserstoffharze sowie zusätzliche Bindemittel, enthalten. Diese Bindemittel haben sich eher als hinderlich zur Erzielung eines wirklich starken Spiegeleffekts erwiesen.

Ohne an eine Theorie gebunden zu sein vermuten die Erfinder, dass bei Nagellackkompositionen die die oben genannten Bindemittel enthalten, diese aufgrund ihrer stärkeren Polarität zumindest teilweise die Effektpigmente benetzen und diese dadurch schlechtere leafing-Eigenschaften aufweisen.

Lösemittel:

Die erfindungsgemäßen Nagellackkompositionen enthalten bevorzugt bestimmte Lösemittel. Als Lösungsmittel können den erfindungsgemäßen Nagellackkompositionen Ethylacetat, Butylacetat, Isopropanol zugesetzt werden.

Bevorzugt enthält die erfindungsgemäße Nagellackkomposition als Lösemittel eine Mischung aus Isopropanol, Ethylacetat und Butylacetat.

Besonders bevorzugt enthält die erfindungsgemäße Nagellackkomposition die Lösemittelmischung aus Isopropanol, Ethylacetat und Butylacetat in einer Menge, die 70 bis 100 Gew.-%, weiter bevorzugt 75 bis 98 Gew.-%, bezogen auf das gesamte Lösemittel der Nagellackkomposition.

Hierbei ist es unerheblich, ob diese bevorzugten Lösemittel über die Bindemittel oder die Effektpigmentdispersion eingetragen werden.

In weiteren bevorzugten Ausführungsformen beträgt bei dieser Lösemittelmischung das Verhältnis von Butylacetat zur Lösemittelmischung 50 bis 99 Gew.-% und besonders bevorzugt 55 bis 98,5 Gew.-%.

Bei einer weiteren besonders bevorzugten Ausführungsform liegt der Anteil von Isopropanol unter 20 Gew.-%, vorzugsweise unter 15 Gew.-%, und weiter bevorzugt unter 10 Gew.-%, jeweils bezogen auf das gesamte Lösemittel.

Zu hohe Anteile an Isopropanol in der erfindungsgemäßen Nagellackkomposition führen zu einem schlechten optischen Erscheinungsbild der Effektpigmente. Vermutlich ist dies auf eine zu schnelle Trocknung der Nagellacke nach ihrer Applikation zurückzuführen.

Bei einer weiteren besonders bevorzugten Ausführungsform umfasst die erfindungsgemäße Nagellackkomposition wenigstens ein oberflächenmodifiziertes Effektpigment basierend auf einem metallischen plättchenförmigen Substrat, wenigstens zwei voneinander verschiedene Kohlenwasserstoffharze sowie die Lösungsmittel Ethylacetat, Butylacetat, Isopropanol.

Die erfindungsgemäßen Nagellackkompositionen sind äußerst einfach auf einem menschlichen oder künstlichen Finger- und/oder Fußnagel applizierbar. Sie zeichnen sich während der Applikation durch einen guten Verlauf aus und bilden nach der anschließenden Trocknung einen homogenen Film auf einem menschlichen oder künstlichen Finger- und/oder Fußnagel.

In bevorzugten Ausführungsformen enthält der erfindungsgemäße Nagellack 50 Gew.-% bis 70 Gew.-%, bevorzugt 55 Gew.-% bis 68 und besonders bevorzugt 57 bis 65 Gew.-%, jeweils bezogen auf das Gewicht des gesamten Nagellacks.

Unterhalb von 55 Gew.-% steigt die Viskosität des Nagellacks zu stark an und die Effektpigmente können sich nicht optimal orientieren, was zu einer Verminderung bis Verlust des Spiegelglanzes führt.

Oberhalb von 70 Gew.-% verringert sich die Viskosität des Nagellacks, was zu einem schlecht kontrollierbaren Auftrag des Nagellacks auf den Fingernagel führt.

Weitere Bestandteile:

Die erfindungsgemäßen Nagellackkompositionen können zusätzlich einen oder mehrere weitere Bestandteile enthalten. Hier sind insbesondere Weichmacher und Antioxidantien zu nennen.

Als Weichmacher können beispielsweise Glycole und ihre Derivate wie beispielsweise Diethylen- glycolethylether, Diethylenglycolmethylether, Diethyleneglycolbutylether oder weiterhin Diethylenglycolhexylether, Ethylenglycolethylether, Ethylenglycolmethylether, Ethyleneglycolbutylether, Ethylenglycolhexylether, Glycolesters, Derivate von Propylenglycol und insbesondere Propylenglycolphenylether, Propylenglycoldiacetate, Dipropylenglycolbutylether, Tripropylenglycolbutylether, Propylenglycolmethylether, Dipropylenglycolethylether, Tripropylenglycolmethylether und Diethylenglycolmethylether, Propylenglycobutylether, oder Mischungen hiervon, verwendet werden.

Weiterhin können als Weichmacher insbesondere Ester von Carbonsäuren, wie beispielsweise von Citraten, insbesondere von Trimethylcitrat, Tributylcitrat, Trimethylacetylcitrat, Tributylacetylcitrat, Triethyl-2-hexylacetylcitrate oder von Phthalatesn, insbesondere Dimethoxyethylphthalat; oder von Phosphaten, insbesonmdere von Tricresylphosphat, Tributylphosphat, Triphenylphosphat, Tributoxyethylphosphate oder von Tartraten, insbesondere Dibuty tartrat; Adipaten, Carbonaten, Sebacaten; Benzylbenzoat, Butylacetylricinoleat, Glycerylacetylricinoleat, Butylglycolat, Kampher, Glyceroltriacetate, N-ethyl-o,p-toluolsulfonamid, Oxyethylen Verbindungen wie beispielsweise Oxyethylenöle, insbesondere pflanzliche Öle, wie beispielsweise Castoröl, Kohlenwasserstofföle und Mischungen hiervon.

Bevorzugte Weichmacher sind insbesondere Kohlenwasserstofföle.

Die Gewichtsanteile der Weichmacher an der gesamten Nagellackkomposition betragen bevorzugt einen Bereich von 0 bis 15 Gew.-%, weiter bevorzugt von 1 bis 10 Gew.-%, und besonders bevorzugt von 5 bis 10 Gew.-%.

Antioxidantien:

Die erfindungsgemäße Nagellackkomposition kann weiterhin ein oder mehrere Antioxidantien enthalten.

Unter "Antioxidantien" werden Verbindungen verstanden, die die Bestandteile des erfindungsgemäßen Nagellacks, insbesondere die Kohlenwasserstoffbindemittel, vor dem Einfluss von Sauerstoff, Hitze, Ozon, und/oder UV-Strahlung schützen. Es können eine oder mehrere derartige Verbindungen verwendet werden.

Beispiele für derartige Verbindungen sind IRGANOX® 1010, IRGANOX® 565, IRGANOX® 1076 (Fa. BASF) oder Schwefel-haltige Antioxidantien wie beispielsweise Zinkdibutyldithiocarbamat (PERKACIT ZDBC.(Fa. Performance additives Italy S.p.A). Bevorzugt werden die Antioxidantien in Mengen aus einem Bereich von 0 bis 5 Gew-%, weiter

bevorzugt aus einem Bereich von 0,05 bis 1 Gew.-%, bezogen auf die gesamte Nagellackkomposition, eingesetzt.

Weitere Additive:

Die erfindungsgemäße Nagellackkomposition kann weiterhin übliche weitere Additive enthalten, wie sie dem Fachmann bekannt sind.

Derartige weitere Additive sind beispielsweise Antiabsetzmittel, Konservierungsstoffe, Öle, Wachse, Radikalfänger, Benetzungsadditive, Dispergierhilfsmittel, Benetzungshilfsmittel, Antischaummittel, Parfüm, Neutralisiermittel, Verdicker, UV-Blocker Feuchthaltemittel, Vitamine, Proteine und Mischungen hiervon.

In weiteren Ausführungsformen enthält die erfindungsgemäße Nagellackkomposition vorzugsweise keine Antiabsetzmittel. Etwaig abgesetztes oberflächenmodifiziertes Effektpigmente lassen sich erstaunlicherweise in aller Regel auch ohne den Zusatz von Antiabsetzmitteln durch einfaches Aufschütteln wieder dispergieren.

Die erfindungsgemäße Nagellackkomposition weist vorzugsweise eine Viskosität von 10 sec bis 16 sec auf, gemessen mit einem DIN Auslaufbecher (DIN 4 mm) gemäß DIN 53211.

In bevorzugten Ausführungsformen enthält die erfindungsgemäße Nagellackkomposition Aluminium-PVD Effektpigmente mit einer mittleren Dicke $h_{50}$ aus einem Bereich von 14 bis 40 nm, bevorzugt aus einem Bereich von 15 bis 35 nm, die mit Phosphorsäurecetylester oder Laurylphosphonsäure als leafing-Additiv beschichtet sind, aromatische Kohlenwasserstoffharze als Bindemittel und eine Mischung aus Isopropanol, Ethylacetat und Butylacetat als Lösemittel, wobei diese Lösemittelmischung 70 bis 100 Gew.-% des gesamten Lösemittels der Nagellackkomposition ausmachen.

Bei einer weiteren bevorzugten Ausführungsform enthält die erfindungsgemäße Nagellackkomposition Aluminium-PVD Effektpigmente mit einer mittleren Dicke $h_{50}$ aus einem Bereich von 14 bis 40 nm, bevorzugt aus einem Bereich von 15 bis 35 nm, sowie wenigstens zwei voneinander verschiedene aromatische Kohlenwasserstoffharze und eine Mischung aus Isopropanol, Ethylacetat und Butylacetat als Lösemittel, wobei diese Lösemittelmischung 70 bis 100 Gew.-% des gesamten Lösemittels der Nagellackkomposition ausmachen. Bevorzugt werden auch hier als leafing-Additiv Phosphorsäurecetylester oder Laurylphosphonsäure verwendet.

Gegenstand der Erfindung ist weiterhin ein Verfahren zur Herstellung der erfindungsgemäßen Nagellackkomposition, umfassend die Schritte

i) Oberflächenmodifizierung des Effektpigments durch ein Additiv in einer Dispersion in einem Lösemittel,
ii) Lösen des Kohlenwasserstoffharzes in einem Lösemittel oder Lösemittelgemisch
iii) Vermischen und Homogenisieren der Dispersion nach i) mit der Bindemittellösung nach ii).

Bevorzugt wird hierbei der Schritt i) gemäß Anspruch 3 ausgeführt.

Das Lösen des Kohlenwasserstoffharzes in einem Lösemittel erfolgt bevorzugt in einem Gemisch aus mindestens zwei, bevorzugt drei Lösemitteln. Besonders bevorzugt wird eine Mischung aus Isopropanol, Ethylacetat und Butylglycol verwendet.

In weiteren bevorzugten Ausführungsformen wird das Lösemittel des Schrittes i) ebenfalls aus Isopropanol, Ethylacetat und Butylglycol oder einer Mischung hiervon bestehen, um nicht weitere, möglicherweise störende Lösemittel in den Nagellack einzutragen.

Weiterhin ist es bevorzugt, bei der Auswahl der metallischen Effektpigmente solche zu wählen, die in einer Paste oder einer Dispersion der bevorzugten Lösemittel Isopropanol, Ethylacetat und Butylglycol vorliegen, da das Lösemittel der Metalleffektpigmentpaste ebenfalls in geringen Mengen in den erfindungsgemäßen Nagellack gelangt, falls nicht aufwendige Umnetzungsschritte eingesetzt werden.

Gegenstand der Erfindung ist ebenfalls ein Verfahren zur Beschichtung eines natürlichen oder künstlichen Fingernagels umfassend die Schritte:

a) Beschichtung des natürlichen oder künstlichen Fingernagels mit einer erfindungsgemäßen Nagellackkomposition und anschließendem Trocknen des Nagellacks,
b) optional darauffolgende Beschichtung des Nagellacks mit einem Klarlack.

Das Aufbringen des Klarlacks erhöht beträchtlich die Abriebbeständigkeit der Nagellackierung. Aufgrund des ausgeprägten leafing-Effektes der Effektpigmente im Nagellack weist dieser naturgemäß eine eher geringe Abriebbeständigkeit auf.

Vor dem Schritt a) kann ebenfalls schon eine Beschichtung des natürlichen oder künstlichen Fingernagels mit einem Klarlack erfolgen, um eine möglichst ebene Oberfläche herzustellen. Diese Vorgehensweise empfiehlt sich, falls die Findernägel eine hohe Rauigkeit aufweisen.

Die nachfolgende Beschichtung mit Klarlack in Schritt b) kann mit dem gleichen oder einem anderen Klarlack wie

der erfindungsgemäße Nagellack durchgeführt werden. Allerdings enthält dieser Klarlack auf keinen Fall Effektpigmente, da diese den gewünschten Effekt des erfindungsgemäßen Nagellacks überlagern würden.

Allerdings kann der Klarlack in Schritt b) konventionelle Farbpigmente oder Farbstoffe enthalten. Gerade in Kombination mit metallischen PVD-Aluminiumpigmenten oder mit dünnen, durch Nassvermahlung hergestellten Aluminiumeffektpigmenten mit einem $h_{50}$-Wert von 20 bis unter 100 nm lassen sich optisch sehr reizvolle Effekte realisieren. Bevorzugt weisen hierbei die mit den Effektpigmenten pigmentierte erfindungsgemäße Nagellackkompositionen nach Schritt a) einen Spiegelglanz auf.

Bei einer weiteren Ausführungsform kann die erfindungsgemäße Nagellackkomposition mit einem niedrig viskosen UV härtenden Klarlack überlackiert werden um die Abriebbeständigkeit der erfindungsgemäßen Nagellackkomposition zu erhöhen.

Bevorzugt können auch lösemittelbasierende Klarlacke verwendet werden. Ohne an eine Theorie gebunden zu sein, basiert der Klarlack bevorzugt auf polaren Bindemitteln, die nur gering mit den Kohlenwasserstoffharzen des erfindungsgemäßen Klarlacks wechselwirken. In besonders bevorzugten Ausführungsformen basierend diese Klarlacke auf Bindemitteln wie Polyvinyl Butyral (PVB), Polyvinylpyrrolidon (PVP) oder Mischungen hiervon.

Weiterhin enthält der Klarlack bevorzugt Lösemittel, die nicht die unpolaren Kohlenwasserstoffharze des erfindungsgemäßen Lacks lösen bzw. anlösen. Beispielsweise kann hierfür Isopropanol verwendet werden. Andernfalls können auch die leafing-Effektpigmente wieder angelöst und in ihrer Orientierung gestört werden, wodurch der Spiegelglanzeffekt gestört wird.

Überraschenderweise weisen die erfindungsgemäßen Klarlacke eine sehr gute Haftungsbeständigkeit auf dem erfindungsgemäßen Nagellack auf.

In einem weiteren Aspekt der Erfindung können die oberflächenmodifizierten Effektpigmente nach Anspruch 1 oder 2 in verwendet werden in weiteren kosmetischen Anwendungen. Hierzu zählen beispielsweise Körperpuder, Gesichtspuder, gepresstes oder loses Puder, Pudercreme, Augenmakeup wie beispielsweise Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haar Gel, Haarwachs, Haarmascara, permanente oder semi-permanente Haarfarben, temporäre Haarfarben oder Hautpflegekompositionen wie Lotionen, Gele, Emulsionen.

Die erfindungsgemäßen oberflächenmodifizierten Effektpigmente werden hierbei mit für die jeweilige Anwendung geeigneten Roh-, Hilfs- und Wirkstoffen kombiniert. Die Gesamtkonzentration erfindungsgemäßen oberflächenmodifizierten Effektpigmente in der kosmetischen Formulierung kann zwischen 0,001 Gew.-% für Rinse-off-Produkte und 40,0 Gew.-% für Leave-on-Produkte, jeweils bezogen auf das Gesamtgewicht der Formulierung, liegen.

Beispiele

Die nachfolgenden Beispiele dienen der näheren Beschreibung der Erfindung und sollen in keinerlei Hinsicht einschränkend sein. Alle Prozentangaben sind Gewichtsprozentangaben. Die Begriffe NFA (nichtflüchtiger Anteil), Festkörperanteil und Feststoffgehalt sind austauschbar verwendbar.

**I Herstellung der erfindungsgemäßen, bevorzugt in einer Nagellackkomposition zu verwendenden, oberflächenmodifizierten Effektpigmente und Herstellung der oberflächenmodifizierten Effektpigmente gemäß Anspruch 1**

Beispiele 1 bis 7:

In einem 1L-Doppelwandreaktor wurden 300 g des Aluminiumeffektpigments METALURE A 41010 AE (Dispersion in Essigsäureethylester, Feststoffgehalt 10%, $D_{50}$ (Horiba LA-930) = 9,5 μm bis 10,5 μm, Fa. ECKART GmbH) in einem Lösemittel gemäß nachstehender Tabelle 2 bei 200 rpm/min dispergiert und auf 40°C erhitzt. Anschließend wurde das Additiv Phosphorsäurecetylester (CAS Nummer: 3539-43-3, Hostaphat CC 100, Fa. Clariant) gemäß nachstehender Tabelle 2, gelöst in 30 g des zum Dispergieren verwendeten Lösemittels, zur Aluminiumeffektpigment-Dispersion zugegeben. Nach 6 Stunden rühren bei 90°C wurde die Mischung abgekühlt und über einen Büchnertrichter filtriert. Es wurden oberflächenmodifizierte Aluminiumeffektpigmente als 5-25%ige Dispersionen erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergaben.

Tabelle 2:

| Beispiel | Lösemittel | Menge Lösemittel [g] | Menge Additiv [g] | NFA [%][1)] |
|---|---|---|---|---|
| 1 | Butylacetat 85/100 | 0 | 3 | 10 |

(fortgesetzt)

| Beispiel | Lösemittel | Menge Lösemittel [g] | Menge Additiv [g] | NFA [%][1)] |
|---|---|---|---|---|
| 2 | Butylacetat 85/100 | 50 | 3 | 9 |
| 3 | Butylacetat 85/100 | 100 | 3 | 11 |
| 4 | Butylacetat 85/100 | 200 | 3 | 7 |
| 5 | Butylacetat 85/100 | 300 | 3 | 23 |
| 6 | Butylacetat 85/100 | 300 | 6 | 21 |
| 7 | Ethylacetat | 200 | 5,4 | 6 |
| [1)] Nichtflüchtiger Anteil des oberflächenmodifizierten Effektpigments. | | | | |

Beispiele 8 bis 12:

In einem 1L-Doppelwandreaktor wurden 300 g des Aluminiumeffektpigments METALURE A 41506 EN (Dispersion in Ethanol, Feststoffgehalt 15%, $D_{50}$ (Horiba LA-930) = 5,5 µm bis 6,5 µm, Fa. ECKART GmbH) in 300 g Lösemittel gemäß nachstehender Tabelle 3 bei 200 rpm/min dispergiert und auf 40°C erhitzt. Anschließend wurde das Additiv gemäß nachstehender Tabelle 3 in 30 g des entsprechenden Lösemittels zur Aluminiumeffektpigment-Dispersion zugegeben. Nach 6 Stunden rühren bei 90°C wurde die Mischung abgekühlt und über einen Büchnertrichter filtriert. Es wurden jeweils oberflächenmodifizierte Aluminiumeffektpigmente als 10-20%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergaben.

Tabelle 3

| Beispiel | Lösemittel | Additiv | Menge [g] | NFA [%] |
|---|---|---|---|---|
| 8 | Butylacetat 85/100 | Hostaphat CC 100[2)] | 3 | 18 |
| 9 | Butylacetat 85/100 | Hostaphat CC 100 | 6 | 15 |
| 10 | Methoxypropanol | Hostaphat CC 100 | 9 | 18 |
| 11 | Methoxypropanol | Laurylphosphonsäure[3)] | 6 | 14 |
| 12 | Butylacetat 85/100 | Laurylphosphonsäure | 6 | 15 |
| [2)] Phosphorsäurecetylester, CAS Nummer: 3539-43-3, Fa. Clariant.<br>[3)] Fa. Rhodia. | | | | |

Beispiel 13 :

In einem 1L-Doppelwandreaktor wurden 300 g des Aluminiumeffektpigments METALURE A 41010 AE (Dispersion in Essigsäureethylester, Feststoffgehalt 10%, $D_{50}$ (Horiba LA-930) = 9,5 µm bis 10,5 µm, Fa. ECKART GmbH) in 50 g Butylacetat 85/100 bei 200 rpm/min dispergiert und auf 80°C erhitzt. Anschließend wurden 3 g des Additivs Phosphorsäurecetylester (CAS Nummer: 3539-43-3, Hostaphat CC 100, Fa. Clariant) in 30 g Butylacetat 85/100 zur Aluminiumeffektpigment-Dispersion zugegeben. Nach 6 Stunden rühren bei 40°C wurde die Mischung abgekühlt und über einen Büchnertrichter filtriert. Es wurde ein oberflächenmodifiziertes Aluminiumeffektpigment als 15%ige Dispersion erhalten, welches nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergab.

Beispiel 14:

In einem 1L-Doppelwandreaktor wurden 300 g des Aluminiumeffektpigments METALURE A 41010 AE (Dispersion in Essigsäureethylester, Feststoffgehalt 10%, $D_{50}$ (Horiba LA-930) = 9,5 µm bis 10,5 µm, Fa. ECKART GmbH) in 50 g Butylacetat 85/100 bei 200 rpm/min dispergiert und auf 60°C erhitzt. Anschließend wurden 3 g des Additives Phosphorsäurecetylester (CAS Nummer: 3539-43-3, Hostaphat CC 100, Fa. Clariant) in 30 g Butylacetat 85/100 zur Aluminiumeffetkpigment-Dispersion zugegeben. Nach 6 Stunden rühren bei 40°C wurde die Mischung abgekühlt und

über einen Büchnertrichter filtriert. Es wurde ein oberflächenmodifiziertes Aluminiumeffektpigment als 10%ige Dispersion erhalten, welches nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergab.

Beispiele 15 bis 18:

In einem 1L-Doppelwandreaktor wurden 300 g Effektpigment gemäß nachstehender Tabelle 4 in 300 g Lösemittel gemäß nachstehender Tabelle 4 bei 200 rpm/min dispergiert und auf 90°C erhitzt. Anschließend wurde das Additiv Phosphorsäurecetylester (CAS Nummer: 3539-43-3, Hostaphat CC 100, Fa. Clariant) in 30 g des entsprechenden Lösemittels zur Effektpigment-Dispersion zugegeben. Nach 6 Stunden rühren bei 40°C wurde die Mischung abgekühlt und über einen Büchnertrichter filtriert. Es wurden jeweils oberflächenmodifizierte Effektpigmente als Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack ergaben, dessen optisches Erscheinungsbild auf das jeweils eingesetzte Effektpigment zurückzuführen war.

Tabelle 4

| Beispiel | Effektpigment | Additiv [g] | Lösemittel | NFA [%] |
|---|---|---|---|---|
| | | | | |
| 15 | SILVERSHINE S 1500[4)] | 3 | Monopropylenglykolmonomethylether[8)] | 35 |
| 17 | SYNCRYSTAL Silk Blue[5)] | 3 | Monopropylenglykolmonomethylether | 72 |
| 17 | METALURE A 31017 AE[6)] | 3 | Butylacetat 85/100 | 12 |
| 18 | METALURE A 31017 AE[6)] | 6 | Butylacetat 85/100 | 12 |

[4)] Aluminiumeffektpigmentpaste, Feststoffgehalt 23 bis 27 %, $D_{50}$ (CILAS 1064) = 12 μm bis 18 μm, Fa. ECKART GmbH.
[5)] Titandioxidbeschichtetes Perlglanzpigment mit blauer Interferenzfarbe, $D_{50}$ (Malvern Mastersizer 2000) = 13 μm, Fa. ECKART GmbH.[67)] Aluminiumeffektpigment, Dispersion in Essigsäureethylester, Feststoffgehalt 10%, $D_{50}$ (Horiba LA-930) = 17 μm, Fa. ECKART GmbH.

Beispiele 19 bis 24:

In einem 1L-Doppelwandreaktor wurden 300 g des Aluminiumeffektpigments METALURE L 55350 AE (Dispersion in Essigsäureethylester, Feststoffgehalt 10%, $D_{50}$ (Horiba LA-930) = 11 μm bis 12 μm, Fa. ECKART GmbH) in 300 g Lösemittel gemäß nachstehender Tabelle 5 bei 200 rpm/min dispergiert und auf 40°C erhitzt. Anschließend wurde das jeweils verwendete Additiv in 30 g Butylacetat 85/100 zur Aluminiumeffektpigment-Dispersion zugegeben. Nach 6 Stunden rühren bei 100°C wurde die Mischung abgekühlt und über einen Büchnertrichter filtriert. Es wurden oberflächenmodifizierte Aluminiumeffektpigmente jeweils als 11-20%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergaben.

Tabelle 5

| Beispiel | Additiv | Lösemittel | Menge [g] | NFA [%] |
|---|---|---|---|---|
| 19 | Laurylphosphonsäure | Butylacetat 85/100 | 3 | 18 |
| 20 | Laurylphosphonsäure | Butylacetat 85/100 | 6 | 18 |
| 21 | Laurylphosphonsäure | Monopropylenglykolmonomethylether | 6 | 18,5 |
| 22 | Hostaphat CS 120[6)] | Butylacetat 85/100 | 6 | 15 |
| 23 | Hostaphat CS 120 | Butylacetat 85/100 | 15 | 15 |
| 24 | Hostaphat CC 100 | Butylacetat 85/100 | 3 | 11,7 |

[6)] Phosphorsäurestearylester, CAS-Nummer: 39471-52-8, Fa. Clariant.

Beispiele 25 bis 31, Vergleichsbeispiel 1:

In einem 1L-Doppelwandreaktor wurden 200 g Silverdream Moonlight 50IL(Feststoffgehalt 50%, $D_{50}$ (CILAS 1064) = 15 µm bis 20 µm, Fa. ECKART GmbH) in 525 g Butylacetat 85/100 bei 200 rpm/min dispergiert und auf 40°C erhitzt. Anschließend wurde das gemäß Tabelle 6 verwendete Additiv in 30 g Butylacetat 85/100 zur Aluminiumeffektpigment-Dispersion zugegeben. Nach 6 Stunden rühren bei 90°C wurde die Mischung abgekühlt und über einen Büchnertrichter filtriert. Es wurden oberflächenmodifizierte Aluminiumeffektpigmente als 45-60%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung bei Verwendung der oberflächenmodifizierten Aluminiumeffektpigmente aus den Beispielen 26 bis 32 einen Nagellack mit spiegelähnlichem Glanz bzw. bei Verwendung des oberflächenmodifizierten Aluminiumeffektpigments aus Vergleichsbeispiel 1 einen Nagellack ohne spiegelähnlichen Glanz, mit matten Aluminium Grauton, ergaben.

Tabelle 6

| Beispiel/ Vergleichsbeispiel | Additiv | Menge [g] | NFA [%] |
|---|---|---|---|
| Beispiel 25 | Hostaphat CC 100 | 2,5 | 50 |
| Beispiel 26 | Hostaphat CC 100 | 5 | 53 |
| Beispiel 27 | Hostaphat CC 100 | 7,5 | 53 |
| Beispiel 28 | Hostaphat CC 100 | 20 | 61 |
| Beispiel 29 | Hostaphat CS 120 | 5 | 55 |
| Beispiel 30 | Hostaphat CS 120 | 10 | 53 |
| Beispiel 31 | Amphisol[7)] | 10 | 46 |
| Vergleichsbeispiel 1 | Hostaphat CS 120 | 20 | 58 |
| [7)] 1-Hexadecanolphosphat, 2,2'-Iminobis[ethanol] 1:1, CAS 69331-39-1, Fa. DSM. | | | |

Vergleichsbeispiele 2 bis 8:

In einem 1L-Doppelwandreaktor wurden 300 g des Aluminiumeffektpigments METALURE L 55350 AE (Dispersion in Essigsäureethylester, Feststoffgehalt 10%, $D_{50}$ (Horiba LA-930) = 11 µm bis 12 µm, Fa. ECKART GmbH) in 300 g Butylacetat 85/100 bei 200 rpm/min dispergiert und auf 100°C erhitzt. Anschließend wurde das gemäß Tabelle 7 verwendete Additiv in 30 g Butylacetat 85/100 zur Aluminiumeffektpigment-Dispersion zugegeben. Nach 6 Stunden rühren bei 40°C wurde die Mischung abgekühlt und über einen Büchnertrichter filtriert. Es wurden oberflächenmodifizierte Aluminiumeffektpigmente jeweils als 10-20%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack ohne spiegelähnlichen Glanz, hingegen einen Nagellack mit matten Aluminium Grauton, ergaben.

Tabelle 7

| Vergleichsbeispiel | Additiv | Menge [g] | NFA [%] |
|---|---|---|---|
| 2 | SilCare Silicone 41M80[8)] | 3 | 18 |
| 3 | SilCare Silicone 41M80 | 15 | 15 |
| 4 | Hostaphat KW 340 D[9)] | 3 | 13 |
| 5 | Hostaphat KW 340 D | 15 | 13 |
| 6 | Hostaphat KL 340 D[10)] | 3 | 16 |
| 7 | Hostaphat KL 340 D | 15 | 14 |
| [8)] INCI: C24-28 Alkyl Dimethicone, CAS-Nummer: 192230-29-8, Fa. Aako BV oder Fa. Clariant.<br>[9)] Mono-, di- und tri-(alkyltetraglycolether)-o-phosphorsäurester, CAS-Nummer: 119415-05-3, Fa. Clariant.<br>[10)] Mono-, di- und tri-(alkyltetraglycolether)-o-phosphorsäureester, CAS-Nummer: 121158-63-2; 121158-61-0; 121158-62-1, Fa. Clariant. | | | |

Vergleichsbeispiele 9 bis 11:

In einem 1L-Doppelwandreaktor wurden 300 g Silverdream Moonlight 50IL (Feststoffgehalt 50%. $D_{50}$ (CILAS 1064) = 15 μm bis 20 μm, Fa. ECKART GmbH), in 470 g Butylacetat 85/100 bei 200 rpm/min dispergiert und auf 90°C erhitzt. Anschließend wurde das gemäß Tabelle 8 verwendete Additiv in 30 g Butylacetat 85/100 zur Aluminiumeffektpigment-Dispersion zugegeben. Nach 6 Stunden rühren bei 40°C wurde die Mischung abgekühlt und über einen Büchnertrichter filtriert. Es wurden oberflächenmodifizierte Aluminiumeffektpigmente als 55-65%ige Dispersion erhalten, welche nach Einarbeitung in den Klarlack gemäß IIa (Pigmentierungshöhe: 1,5 Gew.-%, bezogen auf Gesamtgewicht des Klarlacks), Applikation auf einen künstlichen Fingernagel und anschließender Trocknung einen Nagellack ohne spiegelähnlichen Glanz und/oder ohne Leafing-Effekt ergaben.

Tabelle 8

| Vergleichsbeispiel | Additiv | Menge [g] | NFA [%] |
|---|---|---|---|
| 8 | Hostaphat CK 100[11)] | 7,5 | 59 |
| 9 | Hostaphat CK 100 | 15 | 61 |
| 10 | Hostaphat CK 100 | 30 | 59 |
| 11 | Hostaphat CC 100 | 75 | 57 |
| [11)] Kaliumhexadecylhydrogenphosphat, CAS 19035-79-1, Hostaphat CK 100; Fa. Clariant. | | | |

Vergleichsbeispiel 12:

PVD-Aluminiumpigmentdispersion in Ethylacetat METALURE A-41010 AE, Fa. ECKART GmbH, NFA: 10%, $D_{50}$ = 9,50 μm bis 10,50 μm.

Vergleichsbeispiel 13:

PVD-Aluminiumpigmentdispersion in Ethylacetat METALURE L-55350 AE, Fa. ECKART GmbH, NFA: 10%, $D_{50}$ = 11,00 μm bis 12,00 μm.

Vergleichsbeispiel 14:

PVD-Aluminiumpigmentdispersion in Ethylacetat METALURE A-31017 AE, Fa. ECKART GmbH, NFA: 10%, $D_{50}$ = 9,50 bis 10,50 μm.

Vergleichsbeispiel 15:

Aluminiumpigmentpaste SILVERSHINE S2100, Fa. ECKART GmbH, NFA: 48,0% bis 52,0%, $D_{50}$ = 17,0 μm bis 23,0 μm.

Vergleichsbeispiel 16:

Mischung aus 13 Gew.-% METALURE A-41010 AE, Fa. ECKART GmbH und 0,2 Gew.-% Hostaphat CS 120, Fa. Clariant.

**II Herstellung der erfindungsgemäßen Nagellackkompositionen**

IIa Herstellung des Klarlacks:

In einem geeigneten Rührgefäß wurde eine 70 Gew.-%ige Bindemittellösung F100 hergestellt. Hierzu wurden 30 g Butylacetat 98/100 vorgelegt, unter Rühren und Kühlen (12 °C) mit dem Dissolver Dispermat CNf2 (Fa. Getzmann GmbH) 70 g des Bindemittels Kristalex F100 Hydrocarbon Resin (Fa. Eastman) zugegeben und anschließend 30 Minuten bei 3000 bis 4000 rpm/min nachgerührt.

In einem zweiten geeigneten Rührgefäß wurde eine 60 Gew.-%ige Bindemittellösung 5140 hergestellt, Hierzu wurden 40 g Butylacetat 98/100 vorgelegt, unter Rühren und Kühlen (12 °C) mit dem Dissolver Dispermat CNf2 (Fa. Getzmann GmbH) 60 g des Bindemittels Kristalex 5140 Hydrocarbon Resin (Fa. Eastman) zugegeben und anschließend

30 Minuten bei 3000 bis 4000 rpm/min nachgerührt.

Der nichtflüchtige Anteil (Bindemittelfestkörperanteil) der vorstehend beschriebenen Bindemittellösungen wurde gemäß DIN EN ISO 3251:2008 bestimmt.

Zur Herstellung des Klarlacks wurden die 70 Gew.-%ige Bindemittellösung F100 sowie die 60 Gew.-%ige Bindemittellösung 5140 gemäß nachstehender Tabelle unter Rühren mit 900 rpm/min mit dem Laborrührer IKA RW 20 Digital (Fa. IKA) bei Raumtemperatur zusammengegeben und 5 bis 10 Minuten nachgerührt. Anschließend wurden die Lösungsmittel gemäß jeweiliger nachstehender Tabelle nacheinander unter Rühren mit 600 rpm/min zugegeben.

IIb Herstellung der erfindungsgemäßen Nagellackkompositionen

Zur Herstellung der erfindungsgemäßen Nagellackkompositionen der Beispiele 32 bis 35 wurden 4 g des jeweiligen oberflächenmodifizierten Effektpigments gemäß nachstehenden Tabellen 9, 10 und 11 vorgelegt und 96 g des Klarlacks unter Rühren mit 600 rpm/min zugegeben.

Tabelle 9: Die erfindungsgemäßen Nagellackkompositionen der Beispiele 32 bis 35 wiesen einen Bindemittelfestkörperanteil von 30 Gew.-%, bezogen auf das Gesamtgewicht des Klarlacks, auf.

| **Beispiel** | | **Produktname** | **Einwaage (g)** | **Gewichtsverhältnis** |
|---|---|---|---|---|
| Beispiel 32 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin |

| | | | | |
|---|---|---|---|---|
| | | Kristalex 5140 Hydrocarbon Resin, 60 Gew.-% in Butylacetat | 26,0 | 1 : 1 |
| | | Isopropanol | 5,0 | |
| | | Ethylacetat | 18,7 | |
| | | Butylacetat 98/100 | 28,0 | |
| | | Effektpigment gemäß Beispiel 5, NFA: 23% | 4,0 | |
| Beispiel 33 | Klarlack | Kristalex F100 Hydrocarbon Resin, 70 Gew.-% in Butylacetat | 14,8 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 34,6 | |
| | | Isopropanol | 4,5 | |
| | | Ethylacetat | 19,0 | |
| | | Butylacetat 98/100 | 27,5 | |
| | | Effektpigment gemäß Beispiel 5, NFA: 23% | 4,0 | |
| Beispiel 34 | Klarlack | Kristalex F100 Hydrocarbon Resin, 70 Gew.-% in Butylacetat | 8,9 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 41,6 | |
| | | Isopropanol | 4,5 | |
| | | Ethylacetat | 19,0 | |
| | | Butylacetat 98/100 | 26,0 | |
| | | Effektpigment gemäß Beispiel 5, NFA: 23% | 4,0 | |
| Beispiel 35 | Klarlack | Kristalex F100 Hydrocarbon Resin, 70 Gew.-% in Butylacetat | 4,8 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 46,3 | |
| | | Isopropanol | 4,5 | |
| | | Ethylacetat | 17,9 | |
| | | Butylacetat 98/100 | 26,5 | |
| | | Effektpigment gemäß Beispiel 5, NFA: 23 | 4,0 | |

Tabelle 10: Die erfindungsgemäßen Nagellackkompositionen der Beispiele 36 bis 39 wiesen einen Bindemittelfestkörperanteil von 40 Gew.-%, bezogen auf das Gesamtgewicht des Klarlacks, auf.

| **Beispiel** | | **Produktname** | **Einwaage (g)** | **Gewichtsverhältnis** |
|---|---|---|---|---|
| Beispiel 36 | Klarlack | Kristalex F100 Hydrocarbon Resin, 70 Gew.-% in Butylacetat | 27,9 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 32,5 | |

| | | | | |
|---|---|---|---|---|
| | | Isopropanol | 3,0 | |
| | | Ethylacetat | 11,6 | |
| | | Butylacetat 98/100 | 25,0 | |
| | | Effektpigment gemäß Beispiel 5, NFA: 23% | 4,0 | |
| Beispiel 37 | Klarlack | Kristalex F100 Hydrocarbon Resin, 70 Gew.-% in Butylacetat | 19,8 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 46,3 | |
| | | Isopropanol | 3,1 | |
| | | Ethylacetat | 12,3 | |
| | | Butylacetat 98/100 | 18,5 | |
| | | Effektpigment gemäß Beispiel 5, NFA: 23% | 4,0 | |
| Beispiel 38 | Klarlack | Kristalex F100 Hydrocarbon Resin, 70 Gew.-% in Butylacetat | 11,8 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 55,4 | |
| | | Isopropanol | 2,9 | |
| | | Ethylacetat | 11,9 | |
| | | Butylacetat 98/100 | 20,0 | |
| | | Effektpigment gemäß Beispiel 5, NFA: 23% | 4,0 | |
| Beispiel 39 | Klarlack | Kristalex F100 Hydrocarbon Resin, 70 Gew.-% in Butylacetat | 6,7 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 61,5 | |
| | | Isopropanol | 2,9 | |
| | | Ethylacetat | 11,5 | |
| | | Butylacetat 98/100 | 17,4 | |
| | | Effektpigment gemäß Beispiel 5, NFA: 23% | 4,0 | |

Tabelle 11: Die erfindungsgemäßen Nagellackkompositionen der Beispiele 40 bis 43 wiesen einen Bindemittelfestkörperanteil von 60 Gew.-%, bezogen auf das Gesamtgewicht des Klarlacks, auf.

| Beispiel | | Produktname | Einwaage (g) | Gewichtsverhältnis |
|---|---|---|---|---|
| Beispiel 40 | Klarlack | Kristalex F100 Hydrocarbon Resin, 70 Gew.-% in Butylacetat | 42,9 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 50,0 | |
| | | Isopropanol | 0,6 | |

| | | | | |
|---|---|---|---|---|
| | | Ethylacetat | 2,6 | |
| | | Butylacetat 98/100 | 3,9 | |
| | | Effektpigment gemäß Beispiel 5, NFA: 23% | 4,0 | |
| Beispiel 41 | Klarheit | Kristalex F100 Hydrocarbon Resin, 70 Gew.-% in Butylacetat | 28,6 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 66,7 | |
| | | Isopropanol | 0,4 | |
| | | Ethylacetat | 1,7 | |
| | | Butylacetat 98/100 | 2,6 | |
| | | Effektpigment gemäß Beispiel 5, NFA: 23% | 4,0 | |
| Beispiel 42 | Klarheit | Kristalex F100 Hydrocarbon Resin, 70 Gew.-% in Butylacetat | 17,1 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 80,0 | |
| | | Isopropanol | 0,2 | |
| | | Ethylacetat | 1,0 | |
| | | Butylacetat 98/100 | 1,7 | |
| | | Effektpigment gemäß Beispiel 5, NFA: 23% | 4,0 | |
| Beispiel 43 | Klarheit | Kristalex F100 Hydrocarbon Resin, 70 Gew.-% in Butylacetat | 9,6 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 88,8 | |
| | | Isopropanol | 0,1 | |
| | | Ethylacetat | 0,6 | |
| | | Butylacetat 98/100 | 0,9 | |
| | | Effektpigment gemäß Beispiel 5, NFA: 23% | 4,0 | |

Das optische Erscheinungsbild der erfindungsgemäßen Nagellackkompositionen der Beispiele 32 bis 43 wurde nach Applikation auf einem künstlichen Fingernagel und anschließender Trocknung durch das jeweils eingesetzte oberflächenmodifizierte Effektpigment aus Beispiel 5 bestimmt. Das jeweils eingesetzte oberflächenmodifizierte Effektpigment zeigte in dem gemäß IIa hergestellten Klarlack ein ausgeprägtes Leafing-Verhalten und teilweise einen ausgeprägten Spiegelglanz. Im Hinblick auf das Applikationsverhalten wurde festgestellt, dass sich die erfindungsgemäßen Nagellackkompositionen mit einem Bindemittelfestkörperanteil von 60 Gew.-%, bezogen auf das Gesamtgewicht des Klarlacks, aufgrund ihrer höheren Viskosität schlechter applizieren ließen als die erfindungsgemäßen Nagellackapplikationen mit einem Bindemittelfestkörperanteil von 30 Gew.-% oder 40 Gew.-%, jeweils bezogen auf das Gesamtgewicht des Klarlacks.

Für die erfindungsgemäßen Nagellackkompositionen der Beispiele 44 bis 48 erfolgte die Herstellung des Klarlacks wie vorstehend unter IIa beschrieben. Zur Herstellung der erfindungsgemäßen Nagellackkompositionen der Beispiele 44 bis 48 wurden 4 g des jeweiligen oberflächenmodifizierten Effektpigments gemäß nachstehender Tabelle 12 vorgelegt und 96 g des Klarlacks unter Rühren mit 600 rpm/min zugegeben.

Tabelle 12:

| Pigmentierung, Proben | | Produktname | Einwaage (g) | Gewichtsverhältnis |
|---|---|---|---|---|
| Beispiel 44 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 4,0 | |
| Beispiel 45 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 4,8 | |
| Beispiel 46 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 2,0 | |

| | | | | |
|---|---|---|---|---|
| Beispiel 47 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 7,0 | |
| Beispiel 48 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 9,0 | |

Für die erfindungsgemäßen Nagellackkompositionen der Beispiele 49 bis 64 erfolgte die Herstellung des Klarlacks gemäß Tabelle 13 wie vorstehend unter IIa beschrieben.

Tabelle 13: Klarlack für die Beispiele 49 bis 64 und die Vergleichsbeispiele 24 bis 30

| | **Produktname** | **Einwaage (g)** | **Gewichtsverhältnis** |
|---|---|---|---|
| Klarlack für die Beispiele 49 bis 64 und Vergleichsbeispiele **26 bis 33** | Kristalex F100 Hydrocarbon Resin, 70 Gew.-% in Butylacetat | 19,8 | Kristalex F100 Hydrocarbon Resin : Kristalex 5140 Hydrocarbon Resin 1 : 2 |
| | Kristalex 5140 Hydrocarbon Resin, 60 Gew.-% in Butylacetat | 46,3 | |
| | Isopropanol | 3,1 | |
| | Ethylacetat | 12,3 | |
| | Butylacetat 98/100 | 18,5 | |

Zur Herstellung der erfindungsgemäßen Nagellackkompositionen der Beispiele 49 bis 64 wurde das jeweilige oberflächenmodifizierte Effektpigments gemäß nachstehender Tabelle 14 vorgelegt und der Klarlack aus Tabelle 13 gemäß Tabelle 14 unter Rühren mit 600 rpm/min zugegeben. Die erfindungsgemäßen Nagellackkompositionen der Beispiele 50 bis 66 wiesen einen Bindemittelfestkörperanteil von 40 Gew.-%, bezogen auf das Gesamtgewicht des Klarlacks, auf.

Tabelle 14: Einwaagen an Effektpigmentdispersion und Klarlack für die Beispiele 49 bis 64.

| **Beispiel** | **Nagellackkomposition** | **Einwaage (g)** |
|---|---|---|
| 49 | Effektpigment gemäß Beispiel 1, NFA: 10% | 13,20 |
| | Klarlack aus Tabelle 13 | 86,80 |
| 50 | Effektpigment gemäß Beispiel 15, NFA: 35% | 3,80 |
| | Klarlack aus Tabelle 13 | 96,20 |
| 51 | Effektpigment gemäß Beispiel 14, NFA: 10% | 14,60 |
| | Klarlack aus Tabelle 13 | 85,40 |

(fortgesetzt)

| Beispiel | Nagellackkomposition | Einwaage (g) |
|---|---|---|
| 52 | Effektpigment gemäß Beispiel 11, NFA: 14% | 9,65 |
| | Klarlack aus Tabelle 13 | 90,35 |
| 53 | Effektpigment gemäß Beispiel 12, NFA: 15% | 9,65 |
| | Klarlack aus Tabelle 13 | 90,35 |
| 54 | Effektpigment gemäß Beispiel 10; NFA: 18% | 7,65 |
| | Klarlack aus Tabelle 13 | 92,35 |
| 55 | Effektpigment gemäß Beispiel 21, NFA: 18,5% | 7,36 |
| | Klarlack aus Tabelle 13 | 92,64 |
| 56 | Effektpigment gemäß Beispiel 45, NFA: 11,7% | 11,64 |
| | Klarlack aus Tabelle 13 | 88,36 |
| 57 | Effektpigment gemäß Beispiel 17, NFA: 12% | 11,44 |
| | Klarlack aus Tabelle 13 | 88,56 |
| 58 | Effektpigment gemäß Beispiel 18, NFA: 12% | 11,44 |
| | Klarlack aus Tabelle 13 | 88,56 |
| 59 | Effektpigment gemäß Beispiel 5, NFA: 23% | 6,00 |
| | Klarlack aus Tabelle 13 | 94,00 |
| 60 | Effektpigment gemäß Beispiel 26, NFA: 53% | 2,60 |
| | Klarlack aus Tabelle 13 | 97,40 |
| 61 | Effektpigment gemäß Beispiel 28, NFA: 61% | 2,23 |
| | Klarlack aus Tabelle 13 | 97,70 |
| 62 | Effektpigment gemäß Beispiel 29, NFA: 55% | 2,47 |
| | Klarlack aus Tabelle 13 | 97,53 |
| 63 | Effektpigment gemäß Beispiel 25, NFA: 50% | 2,72 |
| | Klarlack aus Tabelle 13 | 97,28 |
| 64 | Effektpigment gemäß Beispiel 27, NFA: 53% | 2,57 |
| | Klarlack aus Tabelle 13 | 97,43 |

Vergleichsbeispiele

Für die Nagellackkompositionen der Vergleichsbeispiele 17 bis 20 erfolgte die Herstellung des Klarlacks wie vorstehend unter IIa beschrieben. Zur Herstellung der Nagellackkompositionen der Vergleichsbeispiele 18 bis 20 wurden 4 g des jeweiligen oberflächenmodifizierten Effektpigments gemäß nachstehender Tabelle 15 vorgelegt und 96 g des Klarlacks unter Rühren mit 600 rpm/min zugegeben.

Tabelle 15:

| Vergleichs-beispiel | | Produktname | Einwaage (g) | Gewichtsverhältnis |
|---|---|---|---|---|
| Vergleichs-beispiel 17 | Klarlack | Kristalex F100 Hydrocarbon Resin, 70 Gew.-% in Butylacetat | 22,25 | Kristalex F100 Hydrocarbon Resin: Kristalex 5140 Hydrocarbon Resin 1 : 1 |
| | | Kristalex 5140 Hydrocarbon Resin, 60 Gew.-% in Butylacetat | 26,0 | |
| | | Isopropanol | 14,31 | |
| | | Ethylacetat | - | |
| | | Butylacetat 98/100 | 37,44 | |
| | | Effektpigment gemäß Beispiel 5 | 4,0 | |
| Vergleichs-beispiel 18 | Klarlack | Kristalex F100 Hydrocarbon Resin, 70 Gew.-% in Butylacetat | 19,13 | Kristalex F100 Hydrocarbon Resin: Kristalex 5140 Hydrocarbon Resin 1 : 1 |
| | | Kristalex 5140 Hydrocarbon Resin, 60 Gew.-% in Butylacetat | 19,13 | |
| | | Isopropanol | 22,33 | |
| | | Ethylacetat | 39,42 | |
| | | Butylacetat 98/100 | - | |
| | | Effektpigment gemäß Beispiel 5 | 4,0 | |
| Vergleichs-beispiel 19 | Klarlack | Kristalex F100 Hydrocarbon Resin, 70 Gew.-% in Butylacetat | 14,87 | Kristalex F100 Hydrocarbon Resin: Kristalex 5140 Hydrocarbon Resin 1 : 2 |
| | | Kristalex 5140 Hydrocarbon Resin, 60 Gew.-% in Butylacetat | 34,63 | |
| | | Isopropanol | 18,5 | |
| | | Ethylacetat | 32,0 | |
| | | Butylacetat 98/100 | - | |
| | | Effektpigment gemäß Beispiel 5 | 4,0 | |
| Vergleichs-beispiel 20 | Klarlack | Kristalex F100 Hydrocarbon Resin, 70 Gew.-% in Butylacetat | 14,9 | Kristalex F100 Hydrocarbon Resin: Kristalex 5140 Hydrocarbon Resin 1 : 2 |
| | | Kristalex 5140 Hydrocarbon Resin, 60 Gew.-% in Butylacetat | 34,69 | |
| | | Isopropanol | 4,6 | |
| | | Ethylacetat | 15,18 | |
| | | Butylacetat 98/100 | 27,5 | |
| | | Aceton | 3,12 | |
| | | Effektpigment gemäß Beispiel 5 | 4,0 | |

Die Nagellackkompositionen der Vergleichsbeispiele 17 bis 20 zeigten nach der Applikation auf einem künstlichen Fingernagel und anschließender Trocknung ein deutlich schlechteres optisches Erscheinungsbild als die erfindungsgemäßen Nagellackkompositionen, was vermutlich auf das Weglassen eines der Lösungsmittel (Vergleichsbeispiel 17) und/oder einem zu hohen relativen Anteil der Lösemittel Isopropanol und/oder Ethylacetat im Verhältnis zu Butylacetat (Vergleichsbeispiele 18 und 19) zurückzuführen ist. Ferner wirkt sich das Hinzufügen eines zusätzlichen Lösungsmittels (Aceton in Vergleichsbeispiel 20) negativ aus. Die Nagellackapplikationen der Vergleichsbeispiele waren vermutlich aufgrund einer zu schnellen Trocknung weiß angelaufen und trüb. Die genauen Zusammensetzungen der Nagellacke der erfindungsgemäßen Beispiele und der Vergleichsbeispiele können auch Tabelle 18 entnommen werden.

Für die Nagellackkompositionen der Vergleichsbeispiele 21 bis 24 erfolgte die Herstellung des Klarlacks wie vorstehend unter IIa beschrieben. Zur Herstellung der Nagellackkompositionen der Vergleichsbeispiele 21 bis 24 wurden 4 g des jeweiligen oberflächenmodifizierten Effektpigments gemäß nachstehender Tabelle 16 vorgelegt und 96 g des Klarlacks unter Rühren mit 600 rpm/min zugegeben.

Die Nagellackkompositionen der Vergleichsbeispiele 21 bis 24 wiesen einen Bindemittelfestkörperanteil von 20 Gew.-%, bezogen auf das Gesamtgewicht des Klarlacks, auf.

Tabelle 16

| Vergleichs-beispiel | | Produktname | Einwaage (g) | Gewichtsverhältnis |
|---|---|---|---|---|
| Vergleichs-beispiel 21 | Klarlack | Kristalex F100 Hydrocarbon Resin, 70 Gew.-% in Butylacetat | 14,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 16,6 | |
| | | Isopropanol | 6,3 | |
| | | Ethylacetat | 25,1 | |
| | | Butylacetat 98/100 | 37,9 | |
| | | Effektpigment gemäß Beispiel 5, NFA: 23% | 4,0 | |
| Vergleichs-beispiel 22 | Klarlack | Kristalex F100 Hydrocarbon Resin, 70 Gew.-% in Butylacetat | 9,6 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 22,2 | |
| | | Isopropanol | 6,2 | |
| | | Ethylacetat | 24,8 | |
| | | Butylacetat 98/100 | 37,2 | |
| | | Effektpigment gemäß Beispiel 5, NFA: 23% | 4,0 | |
| Vergleichs-beispiel 23 | Klarlack | Kristalex F100 Hydrocarbon Resin, 70 Gew.-% in Butylacetat | 5,7 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 26,6 | |
| | | Isopropanol | 6,1 | |
| | | Ethylacetat | 24,4 | |
| | | Butylacetat 98/100 | 37,0 | |
| | | Effektpigment gemäß Beispiel 5, NFA: 23% | 4,0 | |
| Vergleichs-beispiel 24 | Klarlack | Kristalex F100 Hydrocarbon Resin, 70 Gew.-% in Butylacetat | 3,1 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 29,6 | |
| | | Isopropanol | 6,1 | |
| | | Ethylacetat | 24,5 | |
| | | Butylacetat 98/100 | 36,7 | |
| | | Effektpigment gemäß Beispiel 5, NFA: 23% | 4,0 | |

Die Nagellackkompositionen der Vergleichsbeispiele 21 bis 24 zeigten nach Applikation auf einem künstlichen Fingernagel und anschließender Trocknung ein deutlich schlechteres optisches Erscheinungsbild als die applizierten erfindungsgemäßen Nagellackapplikationen, was vermutlich auf den zu geringen Bindemittelfestkörperanteil von ca. 20 Gew.-%, bezogen auf das Gesamtgewicht des Klarlacks, zurückzuführen ist. Das oberflächenmodifizierte Effektpigment wurde vermutlich durch das im Klarlack vorhandene Lösungsmittel benetzt, weshalb sich das Effektpigment bei bzw. nach der Applikation nicht an der Oberfläche des Klarlacks orientieren konnte.

Zur Herstellung der Nagellackkompositionen der Vergleichsbeispiele 25 bis 30 wurde das jeweilige oberflächenmodifizierte Effektpigments gemäß nachstehender Tabelle 17 vorgelegt und der Klarlack aus Tabelle 13 gemäß Tabelle 17 unter Rühren mit 600 rpm/min zugegeben. Die Nagellackkompositionen der Vergleichsbeispiele 25 bis 30 wiesen einen Bindemittelfestkörperanteil von 40 Gew.-%, bezogen auf das Gesamtgewicht des Klarlacks, auf.

Tabelle 17:

| Vergleichsbeispiel | Nagellackkomposition | Einwaage (g) |
|---|---|---|
| 25 | Effektpigment gemäß Vergleichsbeispiel 10, NFA: 59% | 2,30 |
| | Klarlack aus Tabelle 13 | 97,70 |
| 26 | Effektpigment gemäß Vergleichsbeispiel 1, NFA: 58% | 2,40 |
| | Klarlack aus Tabelle 13 | 97,60 |
| 27 | Effektpigment gemäß Vergleichsbeispiel 15, NFA: 48-52% | 2,70 |
| | Klarlack aus Tabelle 13 | 97,30 |
| 28 | Effektpigment gemäß Vergleichsbeispiel 12, NFA:10% | 13,00 |
| | Klarlack aus Tabelle 13 | 87,00 |
| 29 | Effektpigment gemäß Vergleichsbeispiel 13, NFA: 10% | 13,00 |
| | Klarlack aus Tabelle 13 | 87,00 |
| 30 | Effektpigment gemäß Vergleichsbeispiel 14, NFA: 10% | 13,00 |
| | Klarlack aus Tabelle 13 | 87,00 |

In Tabelle 18 sind die berechneten Kompositionen der erfindungsgemäßen Beispiele und der Vergleichsbeispiele, die sich mit der Variation der Nagellackparameter (bei immer demselben Effektpigment des Beispiels 5) befassen, zusammengefasst.

Tabelle 18:

| Probe | Kristallex F100 | Kristalex 5140 | Isopropanol | Ethylacetat | Butylacetat | weiteres Lösemittel | Pigment gem. Beispiel 5: | Summe | Gesamtgehalt BM in Gew.-% | Gehalt Effektpigment | Gehalt Iso | Gehalt Ethylacetat | Gehalt Butylacetat gesamt |
|---|---|---|---|---|---|---|---|---|---|---|---|---|---|
| Beispiel 32 | 22,3 | 26 | 5 | 18,7 | 28 | | 4 | 104 | 30,0 | 0,88 | 6,96 | 26,02 | 67,02 |
| Beispiel 33 | 14,8 | 34,6 | 4,5 | 19 | 27,5 | | 4 | 104,4 | 29,8 | 0,88 | 6,22 | 26,26 | 67,52 |
| Beispiel 34 | 8,9 | 41,6 | 4,5 | 19 | 26 | | 4 | 104 | 30,0 | 0,88 | 6,26 | 26,43 | 67,31 |
| Beispiel 35 | 4,8 | 46,3 | 4,5 | 17,9 | 26,5 | | 4 | 104 | 29,9 | 0,88 | 6,26 | 24,88 | 68,86 |
| Beispiel 36 | 27,9 | 32,5 | 3 | 11,6 | 25 | | 4 | 104 | 37,5 | 0,88 | 4,68 | 18,11 | 77,21 |
| Beispiel 37 | 19,8 | 46,3 | 3,1 | 12,3 | 18,5 | | 4 | 104 | 40,0 | 0,88 | 5,05 | 20,02 | 74,93 |
| Beispiel 38 | 11,8 | 55,4 | 2,9 | 11,9 | 20 | | 4 | 106 | 39,2 | 0,87 | 4,56 | 18,72 | 76,72 |
| Beispiel 39 | 6,7 | 61,5 | 2,9 | 11,5 | 17,4 | | 4 | 104 | 40,0 | 0,88 | 4,72 | 18,70 | 76,58 |
| Beispiel 40 | 42,9 | 50 | 0,6 | 2,6 | 3,9 | | 4 | 104 | 57,7 | 0,88 | 1,39 | 6,04 | 92,57 |
| Beispiel 41 | 28,6 | 66,7 | 0,4 | 1,7 | 2,6 | | 4 | 104 | 57,7 | 0,88 | 0,93 | 3,95 | 95,12 |
| Beispiel 42 | 17,1 | 80 | 0,2 | 1 | 1,7 | | 4 | 104 | 57,7 | 0,88 | 0,46 | 2,32 | 97,22 |
| Beispiel 43 | 9,6 | 88,8 | 0,1 | 0,6 | 0,9 | | 4 | 104 | 57,7 | 0,88 | 0,23 | 1,39 | 98,38 |
| Beispiel 44 | 22,3 | 26 | 4,7 | 19 | 28 | | 4 | 104 | 30,0 | 0,88 | 6,54 | 26,44 | 67,02 |
| Beispiel 45 | 22,3 | 26 | 4,7 | 19 | 28 | | 4,8 | 104,8 | 29,8 | 1,05 | 6,48 | 26,21 | 67,30 |
| Beispiel 46 | 22,3 | 26 | 4,7 | 19 | 28 | | 2 | 102 | 30,6 | 0,45 | 6,68 | 27,02 | 66,30 |
| Beispiel 47 | 22,3 | 26 | 4,7 | 19 | 28 | | 7 | 107 | 29,2 | 1,50 | 6,34 | 25,61 | 68,05 |
| Beispiel 48 | 22,3 | 26 | 4,7 | 19 | 28 | | 9 | 109 | 28,6 | 1,90 | 6,21 | 25,09 | 68,70 |
| Vergleichsbeispiel 17 | 22,25 | 26 | 14,31 | 0 | 37,44 | | 4 | 104 | 30,0 | 0,88 | 19,90 | **0,00** | 80,10 |
| Vergleichsbeispiel 18 | 19,13 | 19,13 | 22,33 | 39,42 | 0 | | 4 | 104,01 | 23,9 | 0,88 | 28,55 | 50,40 | 21,06 |
| Vergleichsbeispiel 19 | 14,87 | 23,63 | 18,5 | 32 | 0 | | 4 | 93 | 26,4 | 0,99 | 27,41 | 47,41 | 25,18 |
| Vergleichsbeispiel 20 | 14,9 | 34,69 | 4,6 | 15,18 | 27,5 | 3,12 (Aceton) | 4 | 103,99 | 30,0 | 0,88 | 6,40 | 21,13 | 68,12 |
| Vergleichsbeispiel 21 | 14,3 | 16,6 | 6,3 | 25,1 | 37,9 | | 4 | 104,2 | 19,2 | 0,88 | 7,56 | 30,13 | 62,31 |

(fortgesetzt)

| Probe | Kristallex F100 | Kristalex 5140 | Isopropanol | Ethylacetat | Butylacatat | weiteres Lösemittel | Pigment gem. Beispiel 5: | Summe | Gesamtgehalt BM in Gew.-% | Gehalt Effektpigment | Gehalt Iso | Gehalt Ethylacetat | Gehalt Butylacetat gesamt |
|---|---|---|---|---|---|---|---|---|---|---|---|---|---|
| Vergleichsbeispiel 22 | 9,6 | 22,2 | 6,2 | 24,8 | 37,2 | | 4 | 104 | 19,3 | 0,88 | 7,47 | 29,87 | 62,67 |
| Vergleichsbeispiel 23 | 5,7 | 26,6 | 6,1 | 24,4 | 37 | | 4 | 103,8 | 19,2 | 0,89 | 7,36 | 29,42 | 63,22 |
| Vergleichsbeispiel 24 | 3,1 | 29,6 | 6,1 | 24,5 | 36,7 | | 4 | 104 | 19,2 | 0,88 | 7,34 | 29,46 | 63,20 |

**III Charakterisierung der erfindungsgemäßen, bevorzugt in einer Nagellackkomposition zu verwendenden, oberflächenmodifizierten Effektpigmente sowie der oberflächenmodifizierten Effektpigmente**

IIIa Teilchengrößenmessung

Die Größenverteilungskurve der erfindungsgemäßen, bevorzugt in einer Nagellackkomposition zu verwendenden, oberflächenmodifizierten Effektpigmente, der oberflächenmodifizierten Effektpigmente gemäß Anspruch 1 sowie der Pigmente der Vergleichsbeispiele, jeweils basierend auf nichtmetallischen plättchenförmigen Substraten, wurde mit dem Gerät Mastersizer 2000, Fa. Malvern, gemäß Herstellerangaben bestimmt. Hierzu wurden ca. 0,1 g des jeweiligen Pigments als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die Mittelwerte gebildet. Die Auswertung der Streulichtsignale erfolgte dabei nach der Fraunhofer Methode.

Die Größenverteilungskurve der erfindungsgemäßen, bevorzugt in einer Nagellackkomposition zu verwendenden, oberflächenmodifizierten Effektpigmente, der oberflächenmodifizierten Effektpigmente gemäß Anspruch 1 und der Pigmente der Vergleichsbeispiele, jeweils basierend auf metallischen plättchenförmigen Substraten, wurde mit dem Gerät Cilas 1064, Fa. Quantachrome, bzw. dem Gerät Horiba LA-930, Fa. Horiba, jeweils gemäß Herstellerangaben vermessen. Hierzu wurden ca. 50 ml des jeweiligen Pigments in Isopropanol suspendiert, 300 Sekunden im Ultraschallbad (Gerät: Sonorex IK 52, Fa. Bandelin) behandelt und anschließend mittels einer Pasteurpipette in die Probenvorbereitungszelle des Messgerätes gegeben und mehrmals vermessen. Aus den einzelnen Messergebnissen wurden die Mittelwerte gebildet. Die Auswertung der Streulichtsignale erfolgte dabei nach der Fraunhofer Methode.

Unter der mittleren Teilchengröße $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50% der Pigmente einen volumengemittelten Durchmesser aufweisen, der gleich oder kleiner als der angegebene Wert, beispielsweise 20 µm, ist. Entsprechend gibt der $D_{40}$- bzw.$D_{90}$-Wert an, dass 10% bzw. 90%der Pigmente einen volumengemittelten Durchmesser aufweisen, der gleich oder kleiner als der jeweilige Messwert ist.

Der Span ΔD, definiert als $\Delta D = \frac{D_{90} - D_{10}}{D_{50}}$, gibt die Breite der Teilchengrößenverteilung an. Im Hinblick auf das optische Erscheinungsbild der erfindungsgemäßen, bevorzugt in einer Nagellackkomposition zu verwendenden, oberflächenmodifizierten Effektpigmente bzw. der oberflächenmodifizierten Effektpigmente gemäß Anspruch 1 ist ein kleiner Wert von ΔD, d.h. ein enger Span, bevorzugt.

IIIb Bestimmung der mittleren Dicke der metallischen oder nichtmetallischen plättchenförmigen Substrate, Bestimmung der mittleren Gesamtdicke des oberflächenmodifizierten Effektpigments

Die Bestimmung der mittleren Dicken $h_{50}$ der erfindungsgemäßen, bevorzugt in einer Nagellackkomposition zu verwendenden, oberflächenmodifizierten Effektpigmente, die oberflächenmodifizierten Effektpigmente gemäß Anspruch 1 sowie die Pigmente der Vergleichsbeispiele erfolgte gemäß der in der WO 2004/087816 A2 (Seiten 24 und 25) beschriebenen Methode mittels REM.

IIIc Bestimmung des Metalloxidgehaltes der synthetischen Glimmerplättchen

Die Metalloxidgehalte der synthetischen Glimmerplättchen wurden mittels Röntgenfluoreszenzanalyse (RFA) bestimmt. Hierzu wurden die synthetischen Glimmerplättchen in eine Lithiumtetraboratglastablette eingearbeitet, in Festprobenmessbechern fixiert und daraus vermessen. Als Messgerät diente das Gerät Advantix ARL, Fa. Thermo Scientific.

**IV Optische Charakterisierung der erfindungsgemäßen Nagellackkompositionen**

IVa Bestimmung von Glanz (20° Geometrie) und Haze

Zur objektiven Bestimmung des optischen Erscheinungsbilds der erfindungsgemäßen Nagellackkompositionen sowie der Nagellackkompositionen der Vergleichsbeispiele wurde die jeweilige Nagellackkomposition mittels einer Kastenrakel (Filmapplicator Erichsen System Wasag Model 288, Fa. Erichsen) in einer Nassfilmschichtdicke von 110 µm auf Glasplatten appliziert und anschließend bei Raumtemperatur getrocknet. Von den so applizierten Nagellackkompositionen wurde der Glanzwert (20° Geometrie) sowie der Hazewert (Hlog) mit dem Gerät haze gloss (Fa. BYK Gardner) vermessen.

Für die Glanzmessung wurde die 20° Geometrie für hochglänzende Oberflächen herangezogen (Byk-Gardner, Digitaler Katalog "Qualitätskontrolle für Lacke und Kunststoffe", Seite 16). Sowohl der Glanz (20° Geometrie) als auch der Haze (Hlog) wurde an mindestens 5 verschiedenen Stellen der Nagellackapplikation bestimmt. In nachstehender Tabelle 18 sind die hieraus gebildeten Mittelwerte für Glanz (20° Geometrie) und Haze (Hlog) aufgeführt.

**V Ergebnisse:**

In Tabelle 19 werden die Glanz- und Haze-Werte ausgewählter Beispiele und Vergleichsbeispiele aufgeführt. Ferner sind die Pigmentierungshöhen, die Bindemittelkonzentrationen und die Zusammensetzungen der Lösemittel in den Nagellackkompositionen berechnet worden.

Die in Tabelle 19 aufgeführten Glanz- und Haze-Werte sind nicht unabhängig voneinander zu betrachten. Somit kann eine Nagellackkomposition mit einem hohen Haze- sowie gleichzeitig einem niedrigen Glanzwert dennoch einen spiegelähnlichen Effekt aufweisen.

Die erfindungsgemäßen Nagellackkompositionen der Beispiele 48 bis 64 zeigten nach der Applikation ein ausgeprägtes leafing Verhalten bis hin zu einem spiegelähnlichen Effekt. Die optisch hochwertigsten Nagellackapplikationen mit hervorragend ausgebildetem Spiegeleffekt wurden dabei durch die Verwendung von PVD-Pigmenten beschichtet mit Phosphorsäurecetylester (Hostaphat CC 100) erhalten (Beispiele 49, 51, 54,56,57,58 und 59).

Die Nagellackkompositionen der Vergleichsbeispiele 27 bis 30, bei denen kein Additiv verwendet wurde, zeigten hingegen keinen leafing Effekt und zum Teil sogar eine starke Stippenbildung nach der Applikation auf. Dementsprechend schlecht fielen die Glanz- und Hazewerte aus.

Bei Vergleichsbeispiel 25 wurde das Kaliumsalz der Cetylphosphorsäureesters verwendet, was unvorteilhaft ist.

Bei Vergleichsbeispiel 26 enthielt die Applikation Stippen. Vermutlich ist dies auf einen zu hohen Anteil des Additivs bei der Herstellung des beschichteten Metallpigments zurückzuführen.

Tabelle 19: Ergebnisse optischer Untersuchen und wichtigste Kompositionsparameter ausgewählter Beispiele und Vergleichsbeispiele

| Beispiel/ Vergleichs -beispiel | Effektpigmenttyp | Additiv | Additiv-Pigment-Verhältnis in % | Pigmentgehalt [Gew.-%] | Gehalt des bevorzugten 3-Lösemittelgemischs*** bzgl. dem gesamten Lösemittelgehalt | Gehalt Kohlenwasserstoffharz [Gew.-%] | Glanz (20° Geometrie) | Haze (Hlog) |
|---|---|---|---|---|---|---|---|---|
| Beispiel 49 | PVD* | CC 100 | 10 | 1,20 | 71,2 | 57,4 | 624,3 | 1353,9 |
| Beispiel 50 | Pt-$** | CC 100 | 4 | 1,28 | 95,8 | 63,6 | 137,5 | 1344,9 |
| Beispiel 51 | PVD | CC 100 | 10 | 1,33 | 95,8 | 63,6 | 358,4 | 1510,7 |
| Beispiel 52 | PVD | Laurylphosphon -säure | 13,3 | 1,19 | 75,9 | 56,4 | 84,1 | 1289,1 |
| Beispiel 53 | PVD | Laurylphosphon -säure | 13,3 | 1,28 | 78,7 | 59,7 | 130,7 | 1415,0 |
| Beispiel 54 | PVD | CC 100 | 20,0 | 1,15 | 90,8 | 59,7 | 221,2 | 1450,5 |
| Beispiel 55 | PVD | Laurylphosphon -säure | 20,0 | 1,13 | 83,3 | 61,0 | 120,2 | 1383,1 |
| Beispiel 56 | PVD | CC 100 | 10,0 | 1,24 | 84,8 | 61,2 | 149,6 | 1390,4 |
| Beispiel 57 | PVD | CC 100 | 20,0 | 1,14 | 88,5 | 58,4 | 240,1 | 1568,0 |
| Beispiel 58 | PVD | CC 100 | 40,0 | 0,98 | 88,4 | 58,5 | 455,8 | 1496,1 |
| Beispiel 59 | PVD | CC 100 | 10,0 | 1,25 | 88,4 | 58,5 | 176,6 | 1499,3 |
| Beispiel 60 | Pt-$ | CC 100 | 5,0 | 1,31 | 95,1 | 62,1 | 101,0 | 1290,0 |
| Beispiel 61 | Pt-$ | CC 100 | 20,0 | 1,13 | 100,0 | 64,4 | 107,0 | 1317,7 |
| Beispiel 62 | Pt-$ | CS 100 | 5,0 | 1,29 | 100,0 | 64,6 | 125,1 | 1366,0 |
| Beispiel 63 | Pt-$ | CC 100 | 2,5 | 1,33 | 100,0 | 64,5 | 121,1 | 1347,6 |
| Beispiel 64 | Pt-$ | CC 100 | 7,5 | 1,27 | 100,0 | 64,3 | 99,9 | 1289,0 |
| Vergleichsbeispiel 28 | PVD | - | 0 | 1,30 | 100,0 | 36,2 | 101,8 | 121,6 |
| Vergleichsbeispiel 29 | PVD | -- | 0 | 1,30 | 100,0 | 36,2 | 19,2 | 565,6 |
| Vergleichsbeispiel 30 | PVD | -- | 0 | 1,30 | 100,0 | 36,2 | 100,4 | 134,5 |

(fortgesetzt)

| Beispiel/ Vergleichs -beispiel | Effektpigmenttyp | Additiv | Additiv-Pigment-Verhältnis in % | Pigmentgehalt [Gew.-%] | Gehalt des bevorzugten 3-Lösemittelgemischs*** bzgl. dem gesamten Lösemittelgehalt | Gehalt Kohlenwasserstoffharz [Gew.-%] | Glanz (20° Geometrie) | Haze (Hlog) |
|---|---|---|---|---|---|---|---|---|
| Vergleichsbeispiel 27 | Pt-$ | - | 0 | 1,35 | 96,1 | 40,5 | 104,4 | 175,5 |
| Vergleichsbeispiel 25 | Pt-$ | Hostaphat CK 100 | 20 | 1,13 | 100,0 | 64,6 | 73,2 | 1154,9 |
| Vergleichsbeispiel 26 | Pt-$ | CS 120 | 20 | 1,16 | 100,0 | 64,5 | 125,2 | 1363,5 |

* Hier sind alle PVD-Pigmente lediglich mit "PVD" abgekürzt, die weiteren Details entnehme man den entsprechenden vorangegangenen Tabellen.

** Mit "Pt-$" werden in dieser Tabelle alle nassvermahlenen Pigmente mit einer mittleren Dicke von unter 100 nm bezeichnet. Weitere Details entnehme man den entsprechenden vorangegangenen Tabellen.

*** Mischung aus Essigsäureethylester, Butylacetat und Isopropanol.

## Patentansprüche

1. Oberflächenmodifizierte Effektpigmentdispersion umfassend ein optional mit wenigstens einem Metalloxid, Metallhydroxid und/oder Metalloxidhydrat beschichtetes, metallisches plättchenförmiges Substrat, wobei das metallische plättchenförmige Substrat durch PVD-Verfahren hergestellt wird und eine mittlere Dicke $h_{50}$ aus einem Bereich von 13 nm bis 60 nm aufweist und als Ausgangsmaterial (Additiv) zur Oberflächenmodifizierung

   i) Cetylphosphorsäureester oder Cetylphosphorsäure aus einem Bereich von 10 Gew.-% bis 50 Gew.-%, bezogen auf das Gesamtgewicht des optional beschichteten, metallischen plättchenförmigen Substrats, verwendet wird,
   wobei

   das metallische plättchenförmige Substrat ein Aluminiumeffektpigment ist und durch PVD-Verfahren hergestellt wird und einen $D_{50}$-Wert aus einem Bereich von 2,5 µm bis 90 µm aufweist.

2. Oberflächenmodifizierte Effektpigmentdispersion nach Anspruch 1, **dadurch gekennzeichnet,**
   **dass** das metallische plättchenförmige Substrat ein PVD-Aluminiumpigment ist und einen $D_{50}$-Wert aus einem Bereich von 7 µm bis 16 µm und einer mittleren Dicke aus einem Bereich von 14 nm bis 40 nm aufweist.

3. Oberflächenmodifizierte Effektpigmentdispersion nach einem der Ansprüche 1 bis 2, **dadurch gekennzeichnet, dass** das Lösungsmittel der Gruppe bestehend aus Isopropanol, Ethylacetat und Butylglycol oder einer Mischung hiervon, entnommen ist.

4. Oberflächenmodifizierte Effektpigmentdispersion nach einem der Ansprüche 1 bis 2, **dadurch gekennzeichnet, dass** das Lösungsmittel Butylacetat ist.

5. Verfahren zur Herstellung der oberflächenmodifizierte Effektpigmentdispersion nach Anspruch 1, wobei das Verfahren die folgenden Schritte umfasst:

   i. Suspendieren des optional mit wenigstens einem Metalloxid, Metallhydroxid und/oder Metalloxidhydrat beschichteten, metallischen plättchenförmigen Substrats in wenigstens einem Lösemittel,
   ii. Zugabe des Cetylphosphorsäureesters e bei optional erhöhter Temperatur zu der Suspension aus Schritt i. und Rühren der nun erhaltenen Suspension,
   iii. Filtrieren des gemäß Schritt ii. erhaltenen oberflächenmodifizierten Effektpigments.

6. Verfahren nach Anspruch 5,
   **dadurch gekennzeichnet, dass** das Lösemittel des Schrittes i) der Gruppe bestehend aus Isopropanol, Ethylacetat und Butylglycol oder einer Mischung hiervon, entnommen ist.

7. Verfahren nach Anspruch 5,
   **dadurch gekennzeichnet, dass** das Lösemittel Butylacetat ist.

8. Verwendung der oberflächenmodifizierten Effektpigmentdispersion gemäß der Ansprüche 1 bis 4 in Nagellacken.

## Claims

1. A surface-modified effect pigment dispersion comprising a metallic platelet-shaped substrate optionally coated with at least one metal oxide, metal hydroxide, and/or metal oxide hydrate, wherein the metallic platelet-shaped substrate is produced by a PVD process and has an average thickness $h_{50}$ ranging from 13 nm to 60 nm, and as a starting material (additive) for surface modification

   i) cetylphosphoric acid esters or cetylphosphoric acid in a range from 10 wt% to 50 wt%, based on the total weight of the optionally coated metallic platelet-shaped substrate,

   wherein the metallic platelet-shaped substrate is an aluminum effect pigment, is produced by a PVD process, and has a $D_{50}$-value in the range of 2.5 µm to 90 µm.

2. A surface-modified effect pigment dispersion according to claim 1, **characterized in that** the metallic platelet-shaped substrate is a PVD aluminum pigment and has a $D_{50}$value in the range of 7 μm to 16 μm and an average thickness in the range of 14 nm to 40 nm.

3. A surface-modified effect pigment dispersion according to any one of claims 1 through 2, **characterized in that** the solvent is selected from the group consisting of isopropanol, ethyl acetate, and butyl glycol, or a mixture thereof.

4. A surface-modified effect pigment dispersion according to any one of claims 1 through 2, **characterized in that** the solvent is butyl acetate.

5. A method for producing the surface-modified effect pigment dispersion according to claim 1, wherein the method comprises the following steps:

   i. suspending the metallic platelet-shaped substrate-optionally coated with at least one metal oxide, metal hydroxide, and/or metal oxide hydrate-in at least one solvent,
   ii. adding the cetylphosphoric acid ester-optionally at an elevated temperature-to the suspension from step i. and stirring the resulting suspension,
   iii. filtering the surface-modified effect pigment obtained in step ii.

6. The method according to claim 5, **characterized in that** the solvent of step i) is selected from the group consisting of isopropanol, ethyl acetate, and butyl glycol, or a mixture thereof.

7. The method according to claim 5, **characterized in that** the solvent is butyl acetate.

8. Use of the surface-modified effect pigment dispersion according to claims 1 through 4 in nail polishes.

## Revendications

1. Dispersion de pigments à effet à surface modifiée comprenant un substrat métallique en forme de plaquettes, éventuellement recouvert d'au moins un oxyde métallique, un hydroxyde métallique et/ou un hydrate d'oxyde métallique, le substrat métallique en forme de plaquettes étant fabriqué par un procédé PVD et présentant une épaisseur moyenne $h_{50}$ comprise entre 13 nm et 60 nm, et comme matière première (additif) pour la modification de surface i) des esters d'acide cétylphosphorique ou de l'acide cétylphosphorique dans une plage comprise entre 10 % et 50 % en poids, par rapport au poids total du substrat métallique en forme de plaquettes éventuellement revêtu, le substrat métallique en forme de plaquettes étant un pigment à effet à base d'aluminium, fabriqué par un procédé PVD et présentant une valeur $D_{50}$-comprise entre 2,5 μm et 90 μm.

2. Dispersion de pigment à effet à surface modifiée selon la revendication 1, **caractérisée en ce que** le substrat métallique en forme de plaquettes est un pigment d'aluminium obtenu par PVD et présente une valeur $D_{50}$comprise entre 7 μm et 16 μm ainsi qu'une épaisseur moyenne comprise entre 14 nm et 40 nm.

3. Dispersion de pigments à effet à surface modifiée selon l'une des revendications 1 à 2, **caractérisée en ce que** le solvant est choisi parmi le groupe constitué de l'isopropanol, de l'acétate d'éthyle et du butylglycol, ou d'un mélange de ceux-ci.

4. Dispersion de pigments à effet à surface modifiée selon l'une des revendications 1 à 2, **caractérisée en ce que** le solvant est l'acétate de butyle.

5. Procédé de fabrication de la dispersion de pigments à effet à surface modifiée selon la revendication 1, ledit procédé comprenant les étapes suivantes :

   i. la mise en suspension du substrat métallique en forme de plaquettes, éventuellement recouvert d'au moins un oxyde métallique, un hydroxyde métallique et/ou un hydrate d'oxyde métallique, dans au moins un solvant,
   ii. ajouter l'ester d'acide cétylphosphorique, éventuellement à température élevée, à la suspension de l'étape i. et

agiter la suspension ainsi obtenue,
iii. filtrer le pigment à effet à surface modifiée obtenu selon l'étape ii.

**6.** Procédé selon la revendication 5,
**caractérisé en ce que** le solvant de l'étape i) est choisi parmi le groupe constitué de l'isopropanol, de l'acétate d'éthyle et du butylglycol, ou d'un mélange de ceux-ci.

**7.** Procédé selon la revendication 5,
**caractérisé en ce que** le solvant est l'acétate de butyle.

**8.** Utilisation de la dispersion de pigments à effet à surface modifiée selon les revendications 1 à 4 dans des vernis à ongles.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1812518 A2
- EP 2227508 A1
- EP 2318463 A1
- EP 2576702 A1
- EP 1462085 A1
- EP 1299066 A2
- EP 1746913 A2
- EP 1792598 A1
- EP 1796794 A1
- EP 1082952 A1
- JP 2012081236 A
- EP 2248514 A2
- WO 2010086165 A1
- WO 2004087816 A2
- WO 2008077612 A2
- EP 1796794 B2


### In der Beschreibung aufgeführte Nicht-Patentliteratur


- *CHEMICAL ABSTRACTS*, 12645-31-7
- *CHEMICAL ABSTRACTS*, 12751-23-4
- *CHEMICAL ABSTRACTS*, 39471-52-8
- *CHEMICAL ABSTRACTS*, 10483-96-2
- *CHEMICAL ABSTRACTS*, 192230-29-8
- *CHEMICAL ABSTRACTS*, 119415-05-3
- *CHEMICAL ABSTRACTS*, 121158-63-2